# EUROPEAN PATENT APPLICATION

(11) **EP 2 409 989 A1**
(43) Date of publication of application: **25.01.2012**
(21) Application number: 10305795.6
(22) Date of filing: 19.07.2010
(51) Int. Cl.: C07K 16/00, C07K 16/28

(54) **Method to improve glycosylation profile for antibody**

(71) Applicant: International-Drug-Development-Biotech, 69570 Dardilly (FR)
(72) Inventor: Vermot-Desroches, Claudine, 69570, Dardilly (FR)
(74) Representative: Colombet, Alain André

(57) **Abstract**

The present invention relates to the production of recombinant glycoproteins or antibodies that have an improved glycosylation profile and effector functions such as ADCC and/or CDC. The present invention is in particular related to the production of glycoproteins or antibodies having a valuable glycosylation profile, especially a low fucose level and/or a high oligomannose level and /or presence of sialic acid to the glycans.

## Description

**FIELD OF THE INVENTION**

The present invention relates to the production of recombinant glycoproteins or antibodies that have an improved glycosylation profile and effector functions such as ADCC and/or CDC. The present invention is in particular related to the production of glycoproteins or antibodies having a valuable glycosylation profile, especially a low fucose level and/or a high oligomannose level and /or presence of sialic acid to the glycans.

**BACKGROUND OF THE INVENTION**

There is a significant need for cancer, autoimmune or inflammation treatments, particularly for treatment of cancer that has proved refractory to standard cancer treatments, such as surgery, radiation therapy, chemotherapy, and hormonal therapy.

A promising alternative is immunotherapy, in which cancer cells are specifically targeted by cancer antigen-specific antibodies.

Major efforts have been directed at harnessing the specificity of the immune response, for example, hybridoma technology has enabled the development of tumor selective monoclonal antibodies.

In the past few years, the Food and Drug Administration has approved the first MAbs for cancer therapy: Rituxan^{™} (anti-CD20) for non-Hodgkin's Lymphoma and Herceptin^{™} [anti-(c-erb-2/HER-2)] for metastatic breast cancer.

Recently, therapeutic antibodies have been shown to improve overall survival as well as time to disease progression more particularly in a variety of human malignancies, such as breast, colon and haematological cancers.

The term "monoclonal antibody" as used herein refers to an antibody obtained from a population of substantially homogeneous antibodies, i.e., the individual antibodies comprising the population are identical except for possible naturally occurring mutations or alternative post-translational modifications that may be present in minor amounts, whether produced from hydridomas or recombinant DNA techniques.

Antibodies are proteins, which exhibit binding specificity to a specific antigen. Native antibodies are usually heterotetrameric glycoproteins of about 150,000 Daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly intrachain disulfide bridges.

Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light chain variable domain is aligned with the variable domain of the heavy chain.

The term 'variable' refers to the fact that certain portions of the variable domains differ extensively in sequence among antibodies and are responsible for the binding specificity of each particular antibody for its particular antigen. However, the variability is not evenly distributed through the variable domains of antibodies. It is concentrated in three segments called complementarily determining regions (CDRs) both in the light chain and the heavy chain variable domains.

The more highly conserved portions of the variable domains are called the framework regions (FR). The variable domains of native heavy and light chains each comprise four FR regions, largely adopting a β-sheet configuration, connected by three CDRs, which form loops connecting, and in some cases forming part of the β-sheet structure. The CDRs in each chain are held together in close proximity by the FR regions and, with the CDRs from the other chain, contribute to the formation of the antigen binding site of antibodies (Kabat *et al.,* 1991).

The constant domains are not involved directly in binding an antibody to an antigen, but exhibit various effector functions. Depending on the amino acid sequence of the constant region of their heavy chains, antibodies or immunoglobulins can be assigned to different classes. There are five major classes of immunoglobulins: IgA, IgG, IgD, IgE and IgM, and several of these may be further divided into subclasses (isotypes), e.g. IgG1, IgG2, IgG3 and IgG4; IgA1 and IgA2. The heavy chain constant regions that correspond to the different classes of immunoglobulins are called α, δ, ε, γ, and µ, respectively. Of the various human immunoglobulin classes, only IgG1, IgG2, IgG3 and IgM are known to activate complement.

Since their discovery by Köhler and Milstein (1975), MAbs have been intensively engineered to optimize their therapeutic properties. Many technical efforts have been devoted over the last two decades to the generation of second generation MAbs with decreased immunogenicity, better avidity/affinity, and optimized effector functions

The mechanism of action of MAbs is complex and appears to vary for different MAbs. There are multiple mechanisms by which MAbs cause target cell death. These include apoptosis, complement-dependent cytotoxicity (CDC), antibody-dependent cell-mediated cytotoxicity (ADCC) antibody-dependent cell-mediated phagocytosis (ADCP), and inhibition of signal transduction. Cytotoxicity may also be mediated via anti proliferative effects and inhibition of signal transduction.

The most studied is ADCC, which is mediated by natural killer (NK) cells. This involves binding of the Fab portion of an antibody to a specific epitope on a cancer cell and subsequent binding of the Fc portion of the antibody to the Fc receptor on the NK cells. This triggers release of perforin, and granzyme that leads to DNA degradation, induces apoptosis and results in cell death. Among the different receptors for the Fc portion of MAbs, the FcgRIIIa plays a major role in ADCC.

Previous research has shown that a polymorphism of the Fcg RIIIa gene encodes for either a phenylalanine (F) or a valine (V) at amino acid 158. Expression of the valine isoform correlates with increased affinity and binding to MAbs (Rowland *et al.,* 1993; Sapra *et al.,* 2002; Molhoj *et al.,* 2007). Some clinical studies have supported this finding, with greater clinical response to rituximab in patients with non-Hodgkin's lymphoma who display the V/V polymorphism (Cartron *et al.,* 2002; Bruenke *et al. 2005, ,* Bargou *et al.,* 2008).

Today, a wide range of recombinant proteins for therapeutic applications (i.e cancer, inflammatory diseases...) are composed of glycosylated monoclonal antibodies.

Whereas the antigen specificity of antibodies is determined by the antigen-binding Fab portion, the effector functions initiated by antibodies are triggered by the Fc (crystallisable) domain. These effector functions are heavily dependent on the single N-linked, biantennary glycan of the heavy chain, which resides just below the hinge region. This glycan is believed to maintain the two heavy chains of the Fc in an open confirmation required for interactions with activating Fc gamma receptors (FcgammaRs). However, the presence of specific sugar moieties on the glycan has profound implications on Fc effector functions.

Moreover, the oligosaccharide component of protein can affect properties relevant to the efficacy of a therapeutic glycoprotein, including physical stability, resistance to protease attack, interactions with the immune system, pharmacokinetics, and specific biological activity.

Such properties may depend not only on the presence or absence, but also on the specific structures, of oligosaccharides. For example, certain oligosaccharide structures mediate rapid clearance of the glycoprotein from the bloodstream through interactions, with specific carbohydrate binding proteins, while others can be bound by antibodies and trigger undesired immune reactions (Jenkins *et al.,* 1996).

Most antibodies contain carbohydrate structures at conserved positions in the heavy chain constant regions, with each isotype possessing a distinct array of N-linked carbohydrate structures, which variably affect protein assembly, secretion or functional activity. The biological activity of certain G immunoglobulins is dependent on the presence and on the type of glycan structure on the molecule and in particular on its Fc component.

Engineering the Fc region of IgG MAbs to modulate IgG/FcγR interactions is a major goal of current studies on therapeutic antibodies. Recently, site-directed and random mutagenesis as well as domain-swapping, NMR and X-ray crystallography allowed to get detailed insights in the molecular mechanisms that govern IgG/FcγR interactions and to define some of the structural determinants that impact IgG binding to the various FcγR. These different studies have led to the design of Fc antibody variants with improved effector functions, such as FcyRIIIA-dependent ADCC. Biophysical and molecular studies have indicated that several amino-acid residues located in the hinge region between the CH1 and CH2 domains and immediately adjacent to the N-terminus of the CH2 domain of IgG1.

The site-directed mutagenesis of residues of the Fc region of human IgG1 has provided a means for optimizing FcγR-dependent IgG effector functions. Notably, it has allowed the design of Fc antibody variants with an improved binding to activating FcγRIIIA. These variants exhibit also enhanced FcyRIIIA-dependent ADCC (Shields *et al.,* 2001; Lazar *et al.,* 2006).

On the other hand, the sugar chain linked to the CH2 domain at position Asn 297, play a critical role in FcγR binding. On the one hand, a common set of IgG1 residues is involved in binding to all FcγR (I-III), but residues outside this common set, notably at the CH2-CH3 interface, was also identified when FcγRII and FcγRIII interactions with human Fc of IgG1 were studied in details (Shields *et al.,* 2001)*.*

IgG molecules of all human and murine subclasses have an N-oligosaccharide attached to the CH2 domain of each heavy chain (at residue Asn 297 for human IgGs). The general structure of N-linked oligosacchararide on IgG is complex type, characterized by a mannosyl-chitobiose core (Man3-GIcNac2-Asn) with or without bisecting GIcNac/L-Fucose (Fuc) and other chain variants including the presence or absence of Galactose (Gal) and sialic acid.

In addition, oligosaccharides may contain zero (GO), one (GI) or two (G2) Gal. The structure of the attached N-linked carbohydrate may vary considerably, depending on the degree of processing, and can include high-mannose, multiply-branched as well as biantennary complex oligosaccharides.

Typically, there is heterogeneous processing of the core oligosaccharide structures attached at a particular glycosylation site such that even monoclonal antibodies exist as multiple glycoforms. Galactosylation profiles which are variable depending on individuals (human serum IgG1s) have been observed. These differences probably reflect differences in the activity of galactosyl-transferases and other enzymes between the cellular clones of these individuals (Jefferis *et al.* 1990).

It has also become increasingly evident that the carbohydrate composition of the glycan linked to Asn 297 has a profound impact on the binding ability of IgG1, although it is still unclear how this effect takes place. The control of the dynamic of transition between an open and closed conformation of the two CH2 domains is likely to be central.

Although not in direct contact with the FcγR, the carbohydrate attached to the conserved residue Asn 297 on Fc is likely to stabilize the conformation of the FcγR binding epitope on Fc (Radaev *et al.,* 2001). It has been hypothesized that deglycosylation causes a conformational change in the relative orientation of the two CH2 domains such as the Fc transitions from an open to a closed conformation, preventing FcγR binding (Radaev *et al.,* 2001). Analysis of IgG1 glycoforms bearing consecutively truncated oligosaccharides confirmed that removal of sugar residues permits the mutual approach of CH2 domains resulting in the generation of a closed conformation (Krapp *et al.,* 2003).

Studies have been conducted to investigate the function of oligosaccharide residue on antibody biological activities. It has been shown that sialic acid of IgG has no effect on ADCC (Boyd *et al.,* 1995). Several reports have shown that Gal residues enhance ADCC (Kumpel *et al.* 1995). Bisecting GlcNac, which is a beta 1,4-GlcNac residue transferred to a core beta-mannose (Man) residue, has been implicated in biological residue of therapeutic antibodies (Lifely *et al.* 1995; Shields *et al.* 2002) have revealed the effect of fucosylated oligosaccharide on antibody effector functions; the Fuc-deficient IgGI have shown 50-fold increased binding to FcγRIII and enhanced ADCC.

Whereas it has been shown that sialic acid of IgG has no effect on ADCC (Boyd *et al.,* 1995), several reports have shown that Gal residues enhance ADCC (Kumpel *et al.,* 1994). Bisecting GlcNac, which is a beta 4-GlcNac residue transferred to a core beta-mannose (Man) residue, has been implicated in biological residue of therapeutic antibodies (Lifely *et al.,* 1995, Shields *et al.,* 2002) have revealed the effect of fucosylated oligosaccharide on antibody effector functions; the Fuc-deficient IgG1 have shown 50-fold increased binding to FcγRIII and enhanced ADCC.

However the addition of terminal sialic acid to the glycan reduces FcgammaR binding and converts IgG antibodies to anti-inflammatory mediators through the acquisition of novel binding activities. These sialylated IgG Fcs have been demonstrated as important for *in vivo* activity of intravenous immunoglobulin. Instead of binding with Fc gammaRs, sialylated Fcs initiate an anti-inflammatory cascade through the lectin receptor SIGN-R1 or DC-SIGN (Anthony *et al.,* 2010). This leads to upregulated surface expression of the inhibitory FcR, Fc gamma RIIb, on inflammatory cells, thereby attenuating autoantibody-initiated inflammation.

The enhanced ADCC of non-fucosylated forms of therapeutic antibodies through improved FcγRIIIa binding was shown to be inhibited by the fucosylated counterparts. In fact, non-fucosylated therapeutic antibodies, not including the fucosylated forms, exhibit the strongest and most saturable *in vitro* and *ex vivo* ADCC among such antibody variants with improved FcγRIIIa binding as those bearing naturally occurring oligosaccharide heterogeneities and artificial amino acid mutations, even in the presence of plasma IgG.

In order to modify the sugar chain structure of the produced glycoprotein, various methods have been attempted, such as (1) application of an inhibitor against an enzyme relating to the modification of a sugar chain, (2) selection of a cell mutant, (3) introduction of a gene encoding an enzyme relating to the modification of a sugar chain, and the like. Specific examples are described below.

Examples of an inhibitor against an enzyme relating to the modification of a sugar chain includes tunicamycin which selectively inhibits formation of GlcNAc-P-P-Dol which is the first step of the formation of a core oligosaccharide which is a precursor of an N-glycoside-linked sugar chain, castanospermin and W-methyl-1-deoxynojirimycin which are inhibitors of glycosidase I, bromocondulitol which is an inhibitor of glycosidase II, 1-deoxynojirimycin and 1,4-dioxy-1,4-imino-D-mannitol which are inhibitors of mannosidase I, swainsonine which is an inhibitor of mannosidase II, swainsonine which is an inhibitor of mannosidase II and the like. Examples of an inhibitor specific for a glycosyltransferase include deoxy derivatives of substrates against N-acetylglucosamine transferase V (GnTV) and the like. Also it is known that 1-deoxynojirimycin inhibits synthesis of a complex type sugar chain and increases the ration of high mannose type and hybrid type sugar chains (Glycobiology series 2 -Destiny of Sugar Chain in Cell, edited by Katsutaka Nagai, Senichiro Hakomori and Akira Kobata, 1993).

Based on these data, several cell lines have been genetically engineered to produce antibodies containing no or low levels of fucose (Mori *et al,* 2004; Yamane-Ohnuki *et al.,* 2004) to engineer the glycosylation patterns of IgG in order to select therapeutic monoclonal antibodies exhibiting particular profiles of FcγR engagement that could be used in various pathologies..

Umana *et al.* and Davis *et al.* showed that an IgG1 antibody engineered to contain increasing amounts of bisected complex oligosaccharides (bisecting N-acetylglucosamine, GlcNAC) allows triggering a strong ADCC as compared to its parental counterpart (Umana *et al.,* 1999; Davies *et al.,* 2001). Second, a lack of fucose on human IgG1 N-linked oligosaccharides has been shown to improve FcγRIII binding and ADCC.

GLYCART BIOTECHNOLOGY AG (Zurich, CH) has expressed N-acetyl-glucosaminyltransferase III (GnTIII) which catalyzes the addition of the bisecting GlcNac residue to the N-linked oligosaccharide, in a Chinese hamster ovary (CHO) cell line, and showed a greater ADCC of IgG1 antibody produced (WO 99/54342; WO 03/011878; WO 2005/044859).

WO20070166306 is related to the modification of an antibody anti-CD19 containing 60% N-acetylglucosamine bisecting oligosaccharides and 10% non-fucosylated N-acetylglucosamine bisecting oligosaccharides produced in a mammalian human 293T embryonal kidney cells transfected with (i) the cDNA for the anti-CD19 antibody and (ii) the cDNA for the GnTIII enzyme.

Recombinant human IgG1 produced in YB2/0 cells (Shinkawa *et al.,* 2003; Siberil *et al.,* 2006) or in CHO-Lec13 (Shields *et al.,* 2002) which exhibited a low-fucose content or were deficient in fucose as compared to the same IgG1 produced in wild-type CHO cells, showed an enhanced ability to trigger cellular cytotoxicity. By contrast, a correlation between galactose and ADCC was not observed and the content of bisecting GIcNAC only marginally affected ADCC (Shinkawa *et al.,* 2003).

By removing or supplanting fucose from the Fc portion of the antibody, KYOWA HAKKO KOGYO (Tokyo, Japan) has enhanced Fc binding and improved ADCC, and thus the efficacy of the MAb (US 6,946,292).

This improved FcγRIIIA-dependent effector functions of low-fucosylated IgG has been shown to be independent from FcγRIII allelic form (Niwa *et al.,* 2005). Moreover, it has been recently shown that the antigenic density required to induce an efficient ADCC is lower when the IgG has a low content in fucose as compared to a highly fucosylated IgG (Niwa *et al.,* 2005).

The Laboratoire Français du Fractionnement et des Biotechnologies (LFB) (France) showed that the ratio Fuc/Gal in MAb oligosaccharide should be equal or lower than 0.6 to get antibodies with a high ADCC (FR 2 861 080).

By contrast to FcγRIII, it has been first proposed that antibodies produced in CHO-Lec13 cells, thus devoided of fucose, showed only a slight improvement in binding to the soluble immobilized Arg131-FcγRIIA polymorphic form and to the soluble FcγRIIB form, but not to the soluble His131-FcγRIIA polymorphic form (both of them corresponding to the extracellular and transmembrane domains). Since the former receptors have arginine at position 131, it was postulated that fucose may interact directly with the FcγRII at this position or alter the IgG1 conformation so that a slight negative effect on FcγRII binding is induced by its presence (Shields *et al.,* 2002).

The same study suggested that the galactose content does not affect binding to FcγRII. However, we have ourselves compared two anti-RhD MAbs differing through their percentage of fucosylated and galactosylated glycoforms for their ability to engage human and mouse FcγR. We have shown that MAb (YB2/0), a low fucosylated antibody, binds strongly to both activating FcγRIII and inhibitory FcγRIIB, as opposed to its highly fucosylated counterpart, MAb(CHO). No difference of binding to FcγRI between these two antibodies was observed (Siberil *et al.,* 2006).

WO20070166306 is related to the use of avian embryonic derived stem cell lines, named EBx®, for the production of proteins and more specifically glycoproteins such as antibodies that are less fucosylated than with usual CHO cells.

The influence of the presence or the absence of glycan-containing residues on the ability of the antibody to interact with effector molecules (Fc receptors and complement) has been demonstrated. Inhibiting glycosylation of a human IgGI, by culturing in the presence of tunicamycin, causes, for example, a 50-fold decrease in the affinity of this antibody for the FcγRI receptor present on monocytes and macrophages (Leatherbarrow *et al.* 1990).

Binding to the FcγRIII receptor is also affected by the loss of carbohydrates on IgG, since it has been described that a non-glycosylated IgG3 is incapable of inducing lysis of the ADCC type (antibody-dependent cellular cytotoxicity) via the FcγRIII receptor of NK cells (Lund *et al.* 1995). However, beyond the necessary presence of these glycan-containing residues, it is more precisely the heterogeneity of their structure which may result in differences in the ability to initiate effector functions.

Thus, one can expect that appropriate changes in the glycosylation or the purification of adequate glycoforms of human IgG1 produced for therapeutic use will make it possible to prepare antibodies capable of exerting a fine-tuning activating and/or immunoregulatory negative functions.

Likewise, it has been shown that major differences in antibody glycosylation occur between cell lines, and even minor differences are seen for a given cell line grown under different culture conditions including the composition of the culture medium, the cell density, the pH, the oxygenation (Lifely *et al.* 1995; Kumpel *et al.* 1994.)

For therapeutic and economical reasons, there is a large interest in obtaining higher specific antibody activity. One way to obtain large increases in potency, while maintaining a simple production process in cell line and potentially avoiding significant, undesirable side effects, is to enhance the natural, cell-mediated effector functions of MAbs. Consequently, engineering the oligosaccharides of IgGs may yield optimized ADCC which is considered to be a major function of some of the therapeutic antibodies, although antibodies have multiple therapeutic functions (e.g. antigen binding, induction of apoptosis, and CDC).

In general, chimeric and humanized antibodies are prepared using genetic recombination techniques and produced using CHO cells as the host cell. In order to modify the sugar chain structure of the antibodies, various methods have been attempted, say application of an inhibitor against an enzyme relating to the modification of a sugar chain, selection of a CHO cell mutant, or introduction of a gene encoding an enzyme relating to the modification of a sugar chain.

Mammalian cells are the preferred hosts for production of therapeutic glycoproteins, due to their capability to glycosylate proteins in the most compatible form for human applications (Jenkins *et al.,* 1996). Bacteria very rarely glycosylates proteins, and like other type of common hosts, such as yeasts, filamentous fungi, insect and plant cells yield glycosylation patterns associated with rapid clearance from the blood stream.

The Chinese hamster ovary (CHO) cells allow consistent generation of genetically stable, highly productive clonal cell lines. They can be cultured to high densities in simple bioreactors using serum-free media, and permit the development of safe and reproducible bioprocesses. Other commonly used animal cells include baby hamster kidney (BHK) cells, NSO- and SP2/0-mouse myeloma cells. Production from transgenic animals has also been tested (Jenkins *et al.,* 1996).

However, it still remain a need to have alternative methods for producing human monoclonal antibodies in a cell suitable for large-scale production that has not been engineered to express or to silent appropriate glycosyltransferase, and that provides consistent human-type glycosylation with an enhanced cell-mediated effector functions. This is the goal of the instant invention.

**DETAILED DESCRIPTION OF THE INVENTION**

The invention generally relates to the field of recombinant glycoprotein or antibody production. More particularly, the invention relates to the use of wild type rodent, preferably CHO cell lines for the production of glycoproteins such as antibodies.

The invention is useful for the production of monoclonal IgG1 antibody subtype having high cell-mediated cytotoxic activity. The invention relates to the use of such antibodies as a drug to treat cancers and inflammatory diseases.

Effector functions such as CDC and ADCC are effector functions that may be important for the clinical efficacy of MAbs. All of these effector functions are mediated by the antibody Fc region and let some authors to impact on the MAb glycosylation level, especially fucosylation of the Fc region which could have a dramatic influence on the efficacy of an antibody.

This let some authors to modify the conditions of production of the antibodies in the CHO cells by testing the impact of different Fc amino acid variants in order to change the glycosylation profile in an attempt to improve some effector functions such as CDC or ADCC.

On the contrary, the present inventors have evaluated the glycosylation profile of various Fc chimeric variant antibodies of human IgG1 subclass directed against the CD19 antigen produced by the Chinese hamster ovary cells, more particularly CHO dhfr-/-, CHO/DG44 or CHO Easy C cell line, (purchased by ATCC, ECACC or CCT respectively) unmodified or untreated with glycosylation inhibitors.

By analyzing and comparing the structure of the sugar chains of several antibodies produced on wild type CHO cells, chR005-1 Fc0 which is an anti-CD19 antibody with a native human Fc sequence and variant anti-CD19 antibodies chR005-1 having mutated Fc sequences (Fc7, Fc20, Fc24, Fc34), the present inventors found that the variant antibodies produced on wild-type CHO host are in a large proportion antibodies or fragment thereof, carrying a common N-linked oligosaccharide structure of a biantennary-type that comprises long chains with terminal GlcNac that are galactosylated and non-fucosylated.

Moreover, a large proportion of these antibodies have a specific glycosylation profile, especially a low fucose level and/or a high oligomannose level and/or higher level of sialylated glycoforms.

In particular, the transfected cells of the invention, e.g. wild-type CHO cells allow to express a large proportion of antibodies or fragment thereof, carrying a common N-linked oligosaccharide structure of a biantennary-type that comprises long chains with terminal GlcNac that are galactosylated and non-fucosylated and which confer strong ADCC activity to antibodies.

The present inventors have thus also demonstrated that very surprisingly, wild type rodent cells such as CHO cells can be used to produce glycoproteins or antibodies having a low level of fucose.

The inventors have also now demonstrated that surprisingly some Fc variant monoclonal antibody expressed in wild type CHO cells displays a human-like glycosylation pattern.

The inventors also found that a large proportion of Fc variants IgG1antibodies population produced in wild type CHO cells has a common N-linked oligosaccharide structure of a biantennary-type that comprises long chains with terminal GlcNac that are galactosylated. Approximatively at least 20% of IgG1 antibodies population contain the N-linked oligosaccharide structure of biantennary-type that is non-fucosylated, which confers a strong ADCC activity to antibodies.

A first object of the invention is thus the use of mutations within the nucleic acid sequence encoding an IgG Fc region to produce an Fc region having a low fucose level.

In an embodiment, said nucleic acid is used to produce a molecule or an antibody containing said Fc.

In an embodiment, said nucleic acid is used to produce a low level of fucose and/or a high level of oligomannose and/or higher level of sialic acid in Fc in mammal cells, especially rodent cells, preferably CHO cells. In a most preferred embodiment, these cells are wild-type cells.

In an embodiment, the use comprises engineering or using a nucleic acid sequence coding for a variant Fc region wherein this variant region comprises one or several amino acid substitutions at the amino acid positions 243, 292, 300, 305, 326, 333 and 396 of the human IgG Fc region.

The human IgG Fc region may be a region of IgG sub-class. It may be an Fc region of IgG1, IgG2, IgG3 or IgG4. In an embodiment, the Fc region is an IgG1 Fc region.

**First embodiment: Fc7 engineered antibody**

The amino acids of the Fc7 region that are substituted in accordance with the invention may be substituted by any amino acid, the condition being in a first embodiment that the whole set of substituted amino acids is able to confer a low level of fucose and/or a high level of oligomannose and/or higher level of sialic acid according to the invention while increasing an ADCC activity compared to the wild type Fc region. Examples of possible substitutions are given thereafter.

In an embodiment, Lys326 is substituted by Ala.

In an embodiment, Glu333 is substituted by Ala.

In an embodiment, the antibody comprises an Fc comprising substitution at position 326 with Alanine (A) and at position 333 with Alanine (A).

In an embodiment, the antibody comprises an Fc region in which Lys326 is substituted by Ala and Glu333 is substituted by Ala.

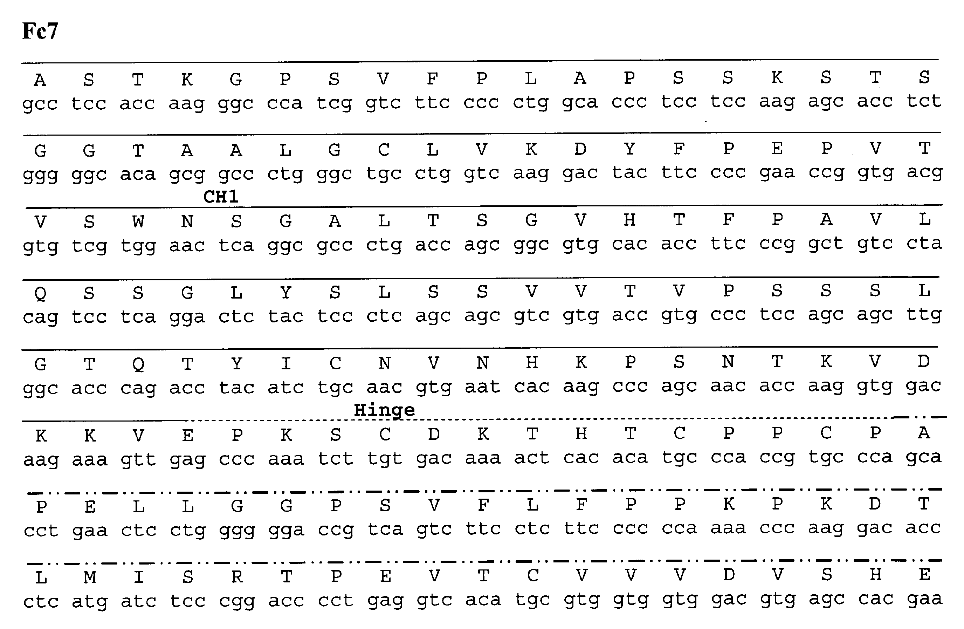

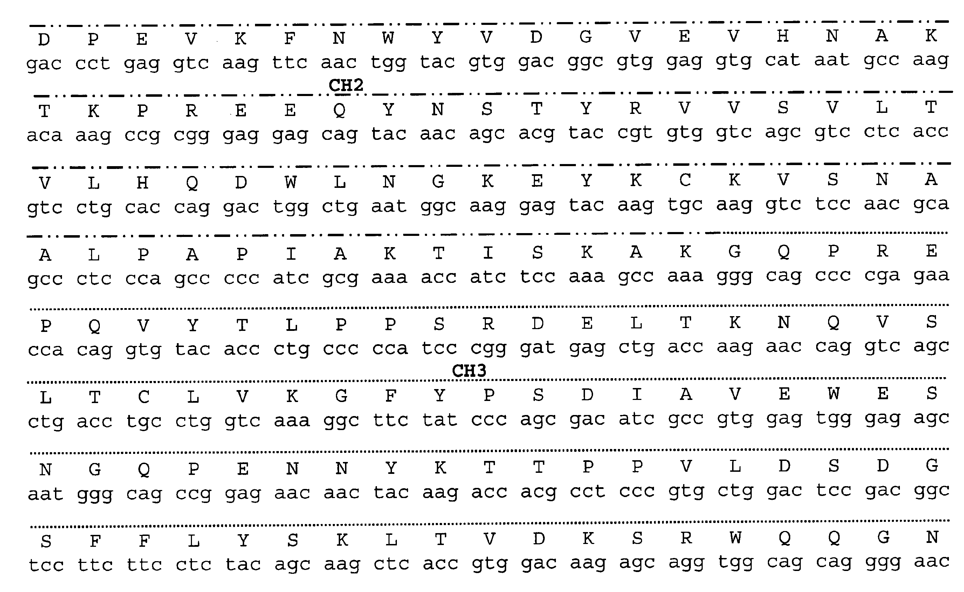

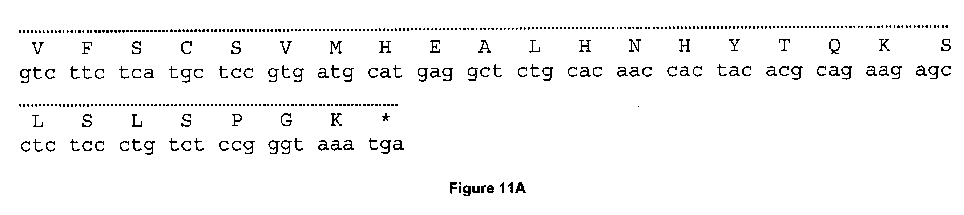

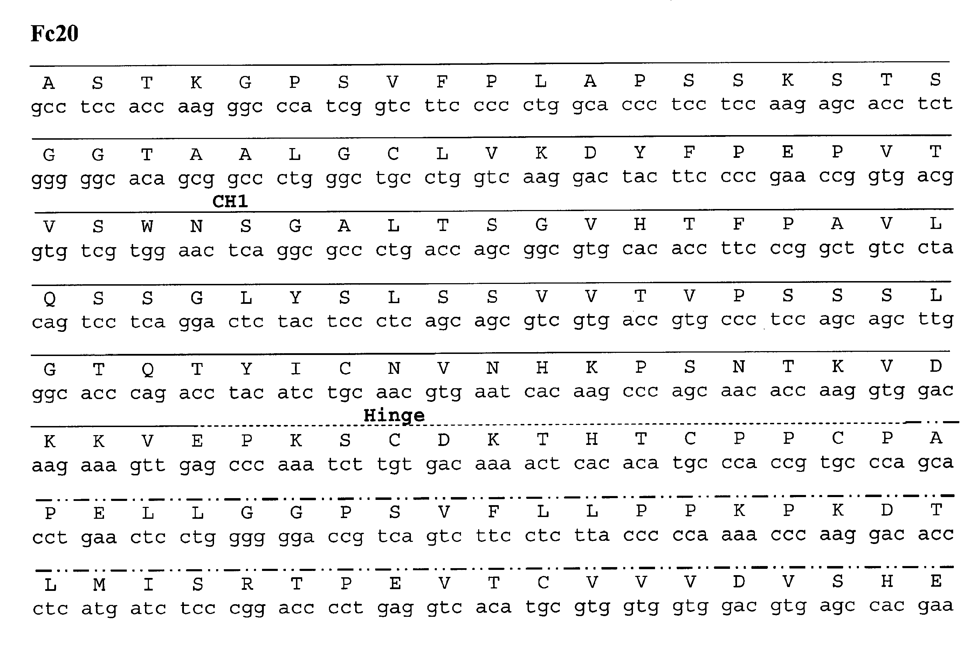

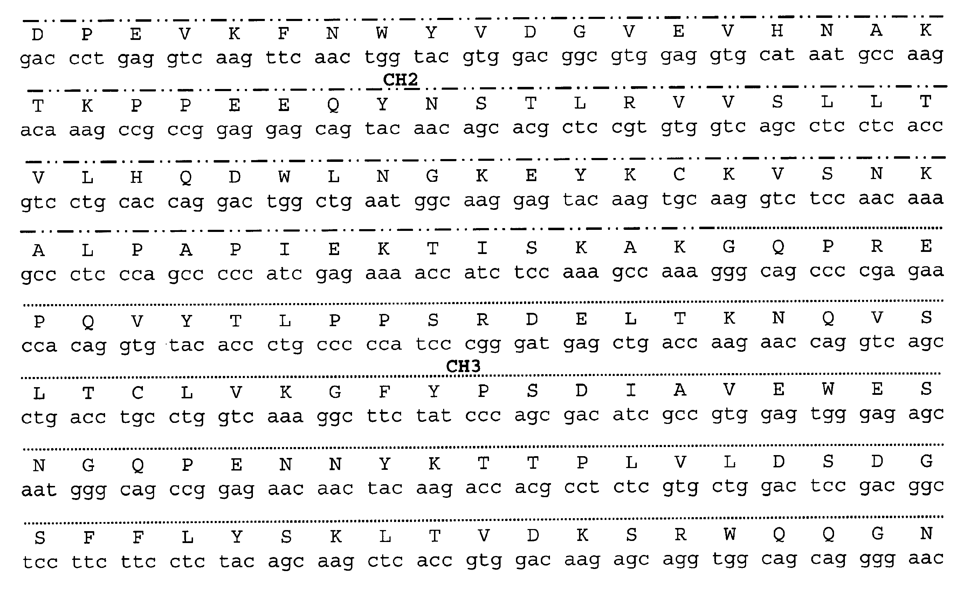

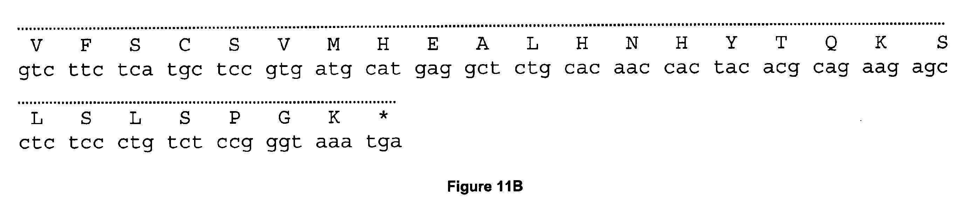

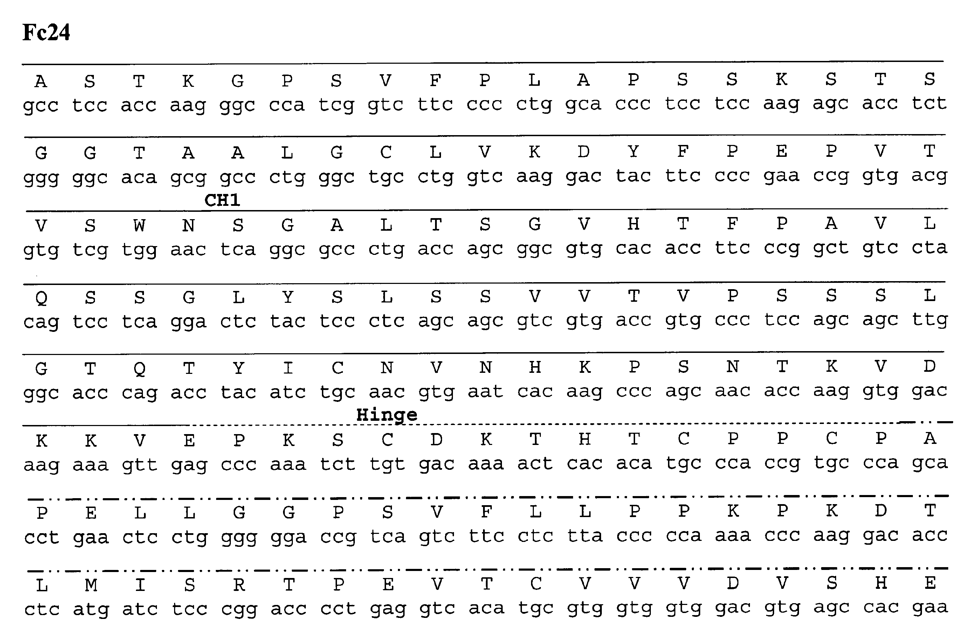

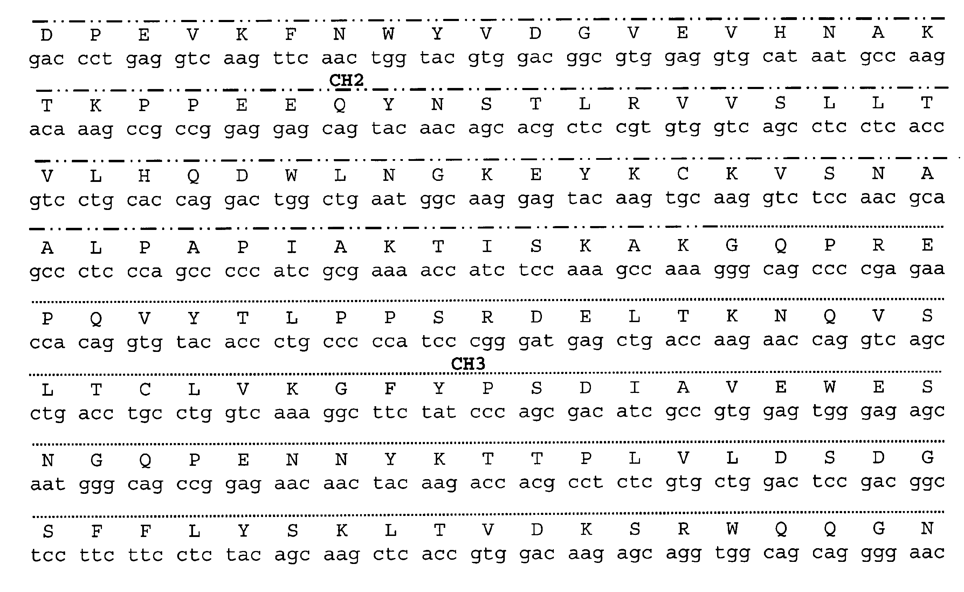

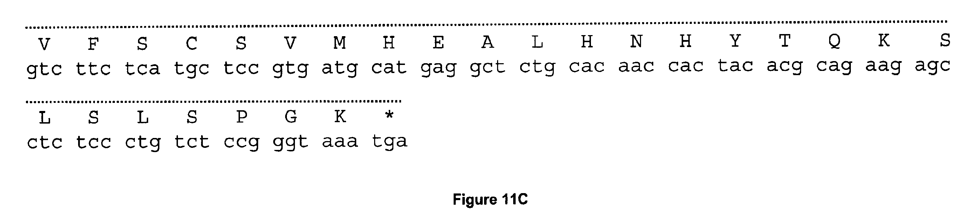

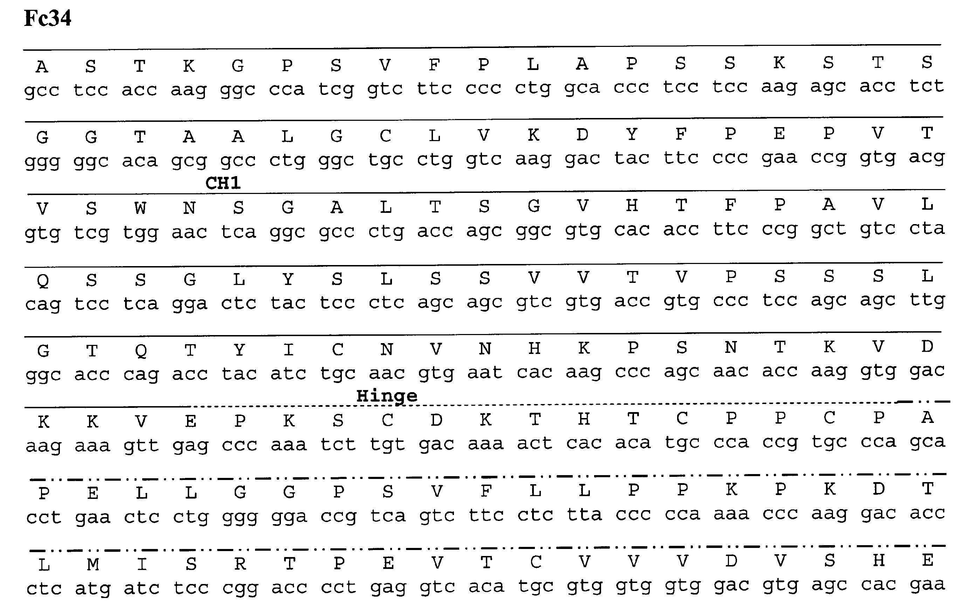

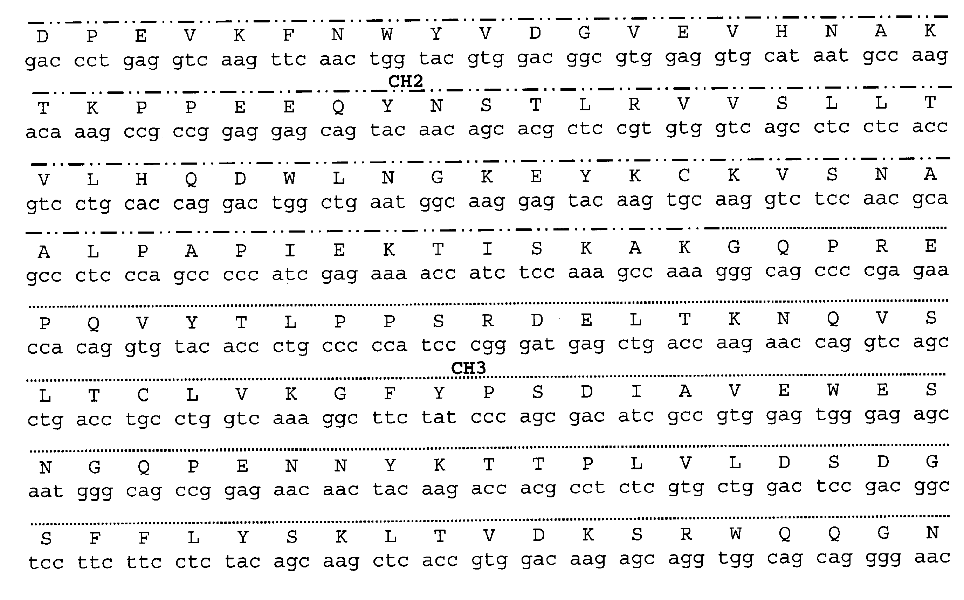

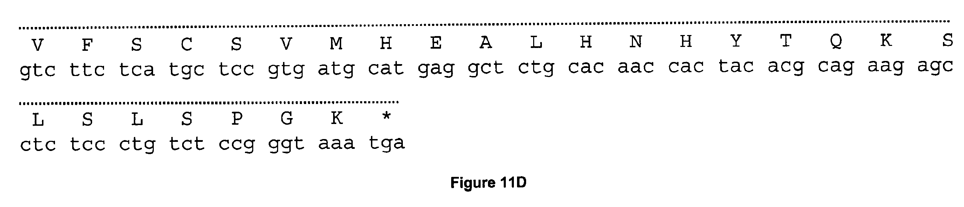

In another embodiment, this Fc7 region has the amino acid sequence depicted on SEQ ID NO: 1 (Fc7, Figure 11). Nucleic acid is depicted on SEQ ID NO: 2.

**Second embodiment: Fc20 engineered antibody**

The amino acids of the Fc20 region that are substituted in accordance with the invention may be substituted by any amino acid, the condition being in a first embodiment that the whole set of substituted amino acids is able to confer a low level of fucose and/or a high level of oligomannose and/or higher level of sialic acid according to the invention while increasing an ADCC activity compared to the wild type Fc region. Examples of possible substitutions are given thereafter.

In an embodiment, Phe243 is substituted by Leu.

In an embodiment, Arg292 is substituted by Pro.

In an embodiment, Tyr300 is substituted by Leu

In an embodiment, Val305 is substituted by Leu.

In an embodiment, Pro396 is substituted by Leu.

In an embodiment, the antibody comprises an Fc comprising substitution at position 243 with Leucine (L), at position 292 with Proline (P), at position 300 with Leucine (L), at position 305 with Leucine (L) and at position 396 with Leucine.

In an embodiment, the antibody comprises an Fc region in which Phe243 is substituted by Leu, Arg292 is substituted by Pro, Tyr300 is substituted by Leu, Val305 is substituted by Leu, and Pro396 is substituted by Leu.

In an embodiment, this Fc region has the amino acid sequence depicted on SEQ ID NO: 3 (Fc20, Figure 11). Nucleic acid sequence coding for this amino acid sequence is SEQ ID NO: 4.

**Third embodiment: Fc24 or Fc34 engineered antibody**

In a third embodiment these amino acids of the Fc24 or Fc34 region are substituted by any amino acid in order that the whole set of substituted amino acids is able to confer a low level of fucose and/or a high level of oligomannose and/or higher level of sialic acid according to the invention while increasing an ADCC activity and a CDC activity compared to the wild type Fc region. Examples of possible substitutions are given thereafter.

In an embodiment, Phe243 is substituted by Leu.

In an embodiment, Arg292 is substituted by Pro.

In an embodiment, Tyr300 is substituted by Leu.

In an embodiment, Val305 is substituted by Leu.

In an embodiment, Lys326 is substituted by Ala.

In an embodiment, Glu333 is substituted by Ala.

In an embodiment, Pro396 is substituted by Leu.

In an embodiment, the antibody comprises an Fc comprising substitution at position 243 with Leucine (L), at position 292 with Proline (P), at position 300 with Leucine (L), at position 305 with Leucine (L), a at position 326 with Alanine (A) and at position 396 with Leucine.

In an embodiment, the antibody comprises an Fc region in which Phe243 is substituted by Leu, Arg292 is substituted by Pro, Tyr300 is substituted by Leu, Val305 is substituted by Leu, Lys326 is substituted by Ala and Pro396 is substituted by Leu.

In an embodiment, this Fc24 region has the amino acid sequence depicted on SEQ ID NO: 5 (Fc24, Figure 11). Nucleic acid sequence coding for this amino acid sequence is SEQ ID NO: 6.

In an embodiment, the antibody comprises an Fc comprising substitution at position 243 with Leucine (L), at position 292 with Proline (P), at position 300 with Leucine (L), at position 305 with Leucine (L), at position 326 with Alanine (A), at position 333 with Alanine (A) and at position 396 with Leucine.

In another embodiment, the antibody comprises an Fc region in which Phe243 is substituted by Leu, Arg292 is substituted by Pro, Tyr300 is substituted by Leu, Val305 is substituted by Leu, Lys326 is substituted by Ala, Glu333 is substituted by Ala, and Pro396 is substituted by Leu.

In an embodiment, this Fc34 region has the amino acid sequence depicted on SEQ ID NO: 7 (Fc34, Figure 11). Nucleic acid sequence coding for this amino acid sequence is SEQ ID NO: 8.

Modifications and changes may be made in the structure of a polypeptide of the present invention and still obtain a molecule having like characteristics. For example, certain amino acids can be substituted for other amino acids in a sequence without appreciable loss of activity. Because it is the interactive capacity and nature of a polypeptide that defines that polypeptide's biological functional activity, certain amino acid sequence substitutions can be made in a polypeptide sequence (or, of course, its underlying DNA coding sequence) and nevertheless obtain a polypeptide with like properties.

In making such changes, the hydropathic index of amino acids can be considered. The importance of the hydropathic amino acid index in conferring interactive biologic function on a polypeptide is generally understood in the art (Kyte *et al.* 1982). It is known that certain amino acids can be substituted for other amino acids having a similar hydropathic index or score and still result in a polypeptide with similar biological activity. Each amino acid has been assigned a hydropathic index on the basis of its hydrophobicity and charge characteristics.

It is believed that the relative hydropathic character of the amino acid determines the secondary structure of the resultant polypeptide, which in turn defines the interaction of the polypeptide with other molecules, for example, enzymes, substrates, receptors, antibodies, antigens, and the like. It is known in the art that an amino acid may be substituted by another amino acid having a similar hydropathic index and still obtain a biologically functionally equivalent polypeptide. In such changes, the substitution of amino acids whose hydropathic indices are within ±2 is preferred, those which are within ±1 are particularly preferred, and those within +0.5 are even more particularly preferred.

Substitution of like amino acids can also be made on the basis of hydrophilicity, particularly where the biologically functionally equivalent peptide or polypeptide thereby created is intented for use in immunological embodiments. U.S. Patent 4,554,101, incorporated herein by reference, states that the greatest local average hydrophilicity of a polypeptide, as governed by the hydrophilicity of its adjacent amino acids, correlate with its immunogenicity and antigenicity, i.e. with a biological property of the polypeptide.

As detailed in U.S. Patent 4,554,101, the following hydrophilicity values have been assigned to amino acid residues: arginine (+3.0); lysine (+3.0); aspartate (+3.0 ±1); glutamate (+3.0 ±1); serine (+0.3); asparagine (+0.2); glutamine (+0.2); glycine (0); proline (-0.5 +1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophility value and still obtain a biologically equivalent, and in particular, an immunologically equivalent, polypeptide. In such changes, the substitution of amino acids whose hydrophilicity values are within ±2 is preferred, those which are within ± 1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like.

| **Amino Acid** | **Index** | **Amino Acid** | **Index** |
|---|---|---|---|
| isoleucine L | (+4,5) | tryptophan W | (-0,9) |
| valine V | (+4,2) | tyrosine Y | (-1,3) |
| leucine L | (+3,8) | proline P | (-1,6) |
| phenylalanine | (+2,8) | histidine H | (-3,2) |
| cysteine C | (+2,5) | glutamate E | (-3,5) |
| methionine M | (+1,9) | glutamine Q | (-3,5) |
| alanine A | (+1,8) | aspartate D | (-3,5) |
| glycine G | (-0,4) | asparagine N | (-3,5) |
| threonine T | (-0,7) | lysine K | (-3,9) |
| serine S | (-0,8) | arginine R | (-4,5) |

Amino acid substitution may be chosen or selected differently. Possible substitutions have been documented in WO99/51642, WO2007024249 and WO2007106707.

In an embodiment, Phe243 is substituted by an amino acid chosen among Leu, Trp, Tyr, Arg and Gln. Preferably, Phe243 is substituted by Leu.

In an embodiment, Arg292 is substituted by an amino acid chosen among Gly and Pro. Preferably, Arg292 is substituted by Pro.

In an embodiment, Tyr300 is substituted by an amino acid chosen among Lys, Phe, Leu and Ile. Preferably, Tyr300 is substituted by Leu.

In an embodiment, Val305 is substituted by Leu and Ile. Preferably, Val305 is substituted by Leu.

In an embodiment, Lys326 is substituted by an amino acid chosen among Val, Glu, Ala, Gly, Asp, Met, Ser, Asn and Trp. Preferably, Lys326 is substituted by Ala.

In an embodiment, Glu333 is substituted by an amino acid chosen among Val, Gly, Ala, Gln, Asp, Asn, Lys, Arg and Ser. Preferably, Glu333 is substituted by Ala.

In an embodiment, Pro396 is substituted by Leu.

In a very surprising and valuable embodiment, the antibodies have a low fucose level. This means that among a antibody population produced in these cells, e.g. wild-type CHO, the proportion of non-fucosylated antibodies represent approximately at least 40%, preferably approximately at least 60%, more preferably approximately at least 80% of the antibodies or higher.

In still a very surprising and valuable embodiment, the antibodies have a high oligomannose level. This means that among a recombinant antibody population produced in these cells, e.g. wild-type CHO the proportion of antibodies featured by a higher level of oligomannoses represent approximately at least 20%, preferably approximately at least 30%, more preferably approximately at least 40%, still more preferably approximately at least 50% of the antibodies or higher.

In still a very surprising and valuable embodiment, among a recombinant antibody population produced in these cells, e.g. wild-type CHO, the proportion of antibodies featured by a higher level of sialylated glycoforms represent approximately at least 1.5%, preferably approximately at least 2.5%, more preferably approximately at least 5% of the antibodies or higher.

In a preferred embodiment, the antibodies of the invention combine three of these features, especially low fucose level and high oligomannose level and/or presence of sialic acid.

In particular, the transfected cells of the invention, e.g. wild-type CHO cells allow to express a large proportion of antibodies or fragment thereof, carrying a common N-linked oligosaccharide structure of a biantennary-type that comprises long chains with terminal GlcNac that are galactosylated and non-fucosylated and which confer strong ADCC activity to antibodies.

In an embodiment, the anti-CD19 antibody having a specific glycosylation profile according to the invention, especially a low fucose level and/or a high oligomannose level and/or presence of sialic acid is produced or expressed, or is as produced or expressed, in mammal cells, preferably wild-type mammal cells.

In another embodiment, the Fc region has a N-linked oligosaccharide structure of a biantennary-type that comprises long chains with terminal GlcNac that are galactosylated.

In an embodiment, the Fc region is used to produce an antibody containing this Fc region.

By way of example, an anti-CD19 antibody may be produced.

In an embodiment, the antibody is specific for, or recognizes, a non-internalizing epitope on the CD19 antigen. The applicant has developed a murine anti-CD19 antibody called mR005-1, whose variable regions have been sequences and the CDRs identified. The present invention thus includes as preferred embodiments the use of the variable regions or the CDRs derived from mR005-1.

In a preferred embodiment, the anti-CD19 antibody mR005-1 of the invention comprises the following CDRs:

| | **SEQ ID** **N°:** | **Sequence** **IMGT ®** | **SEQ ID** **N**°: | **Sequence** **Kabat ®** | **SEQ ID** **N°:** | **Sequence** **(Common numbering system)** |
|---|---|---|---|---|---|---|
| **VH mR005-1** | | | | | | |
| ***CDR1*** | 9 | **GYAFSSYW** | 15 | **SYW**VN | 20 | **SSYW** |
| ***CDR2*** | 10 | **IYPGDGDT** | 16 | Q**IYPGDGDT**NYNGKFKG | 10 | **IYPGDGDT** |
| ***CDR3*** | 11 | **ARSITTVVGCAMDY** | 17 | **SITTVVGCAMDY** | 17 | **SITTVVGCAMDY** |

| **VL mR005-1** | | | | | | |
|---|---|---|---|---|---|---|
| ***CDR1*** | 12 | **DHINNW** | 18 | KAS**DHINNW**LA | 12 | **DHINNW** |
| ***CDR2*** | 13 | **GAT** | 19 | **GAT**TLET | 13 | **GAT** |
| ***CDR3*** | 14 | **QQSWNTPWT** | 14 | **QQSWNTPWT** | 14 | **QQSWNTPWT** |

By definition, these CDRs include variant CDRs, by deletion, substitution or addition of one or more amino acid(s), which variant keeps the specificity of the original CDR. The common numbering system provides for a CDR definition having the shortest amino acid sequences or the minimal CDR definition.

In an embodiment, the anti-CD19 antibody comprises the VH and VL of R005-1. The nucleotide and amino acids sequences of the murine MAbs R005-1 (A): V_{H} (B): VL are shown as one-letter codes.

| | Amino acid | Nucleic acid | Amino acid | Nucleic acid |
|---|---|---|---|---|
| | sequence VH | sequence VH | sequence VL | sequence VL |
| R005-1 | SEQ ID NO: 21 | SEQ ID NO: 22 | SEQ ID NO: 23 | SEQ ID NO: 24 |

Another object of the invention is a method to produce an Fc having a low fucose level, comprising engineering one or several variant IgG Fc region(s) comprising one or several mutations, measuring the fucose level present on the Fc region and recovering Fc region(s) having a low fucose level.

Another object of the invention is a method to produce an Fc having a high oligomannose level, comprising engineering one or several variant IgG Fc region(s) comprising one or several mutations, measuring the oligomannose level present on the Fc region and recovering Fc region(s) having a high oligomannose level.

Another object of the invention is a method to produce an Fc having high level of sialylated glycoforms, comprising engineering one or several variant IgG Fc region(s) comprising one or several mutations, measuring the sialylated glycoform level present on the Fc region and recovering Fc region(s) having a high sialylated glycoform level.

Still another object is such a method to produce an Fc having a low fucose level, a high oligomannose level and a high sialylated glycoform level, comprising engineering one or several variant IgG Fc region(s) comprising one or several mutations, measuring the levels for the three criteria present on the Fc region and recovering Fc region(s) having the required levels.

By recovering Fc region having one of these criteria, any combination thereof and preferably the three criteria, it is meant the recovering of at least a population comprising a large proportion of Fc having the criteria.

In an embodiment, the method comprises the engineering of a nucleic acid encoding the variant Fc region, the cloning of this nucleic acid in an expression vector, the transfection of mammal cells with this expression vector, the recovery of the Fc region having the criteria.

In an embodiment, the mammal cells are rodent cells, preferably CHO cells. In a most preferred embodiment, these cells are wild-type cells.

In an embodiment, upon engineering the mutation in the Fc region, one selects one or several amino acid substitutions at the amino acid positions 243, 292, 300, 305, 326, 333 and 396 of the human IgG Fc region.

In an embodiment, one recovers an Fc region or Fc regions having a fucose level according to the invention.

In an embodiment, one recovers an Fc region or Fc regions having a low fucose level and/or a high level of oligomannose according to the invention.

In an embodiment, one recovers an Fc region or Fc regions having a low fucose level and/or a high level of oligomannose and/or a higher level of sialic acid according to the invention.

In an embodiment, the Fc which is produced is part of a molecule containing this Fc. In an embodiment, the molecule is an antibody. One then recovers the antibody or the antibodies having a fucose level according to the invention. In an embodiment, one recovers the antibody or the antibodies having a low fucose level and/or a high level of oligomannose and/ or a higher level of sialic acid according to the invention.and preferably an ADCC and/or CDC function.

Another object of the invention is a molecule or an antibody obtained by performing the method of the invention.

In the present invention, the terms "cell line" and "cells" will be used indistinctly.

In the present invention, the terms "wild type cell line" means without mutagenesis on glycosylation pathways.

In a preferred embodiment, "rodent" refer to any animal of the taxonomix order.

The present invention provides rodent mammalian cells, preferably an ovarian hamster cells. As cells that may be used, one may mention, without limitation.

The host cells which contain the coding sequence and which express the biologically active gene products (i.e protein of interest, selectable marker, ...) may be identified by at least different general approaches; (a) DNA-DNA or DNA-RNA hybridization; (b) resistance to antibiotics, (c) the presence of membranous Ig at the cell surface; (d) assessing the level of transcription as measured by the expression of the respective mRNA transcripts in the wild type CHO cell; and (e) detection of the gene product as measured by immuno-assay or (f) by its biological activity.

Accordingly, the term "nucleic acid vector" or "plasmid vector" as used herein refers to a natural or synthetic single or double stranded plasmid or viral nucleic acid molecule, or any other nucleic acid molecule that can be transfected or transformed into cells and replicate independently of, or within, the host cell genome. A nucleic acid can be inserted into a vector by cutting the vector with restriction enzymes and ligating the pieces together.

Preferably, the protein of interest is a monoclonal antibody, preferably a human monoclonal antibody, or an altered antibody, and the wild type cell of the invention are co-transfected with a vector capable of expressing the light chain of the antibody and a vector capable of expressing the heavy chain of the antibody or with at least one expression vector.

It should be recognized, however, that the choice of a suitable expression vector and the combination of functional elements therein depends upon multiple factors including for example the type of protein to be expressed.

Representative examples of expression vectors include, for example, bacterial plasmid vectors including expression and cloning vectors. As vectors that may be used, one may mention without limitation: pcDNA3.3, pOptiVEC, pFUSE, pMONO, pSPORT1, pcDV1, pCDNA3, pCDNA1, pRc/CMV, pSEC, pMCMVHE, pRSV, pHCMVHE, pMCMV, pHCMV, pEµMCMV.

Accordingly, the expression vectors of the invention are stably incorporated into the chromosomal DNA of the CHO cell. Following the introduction of foreign DNA, engineered cells may be allowed to grow for 1-2 days in an enriched media, and then are switched to a selective media.

The expression vectors described herein can be introduced into wild type CHO cells by a variety of methods.

In particular, standard transfection procedures, well-known from the man skilled in the art may be carried out, such as calcium phosphate precipitation, DEAE- Dextran mediated transfection, adenoviral or retroviral infection, electroporation, nucleofection (AMAXA® Nucleofector® Technology, Lonza), liposome-mediated transfection (using lipofectin® or lipofectamine® technology for example) or microinjection.

"Co-transfection" means the process of transfecting CHO cell with more than one expression vector. When the cell has been co-transfected with an expression vector capable of expressing the light chain of the antibody and a vector capable of expressing the heavy chain of the antibody, the vectors preferably contain independently selectable markers. When a single expression vector is capable of expressing the light chain of the antibody and the heavy chain of the antibody, the vector preferably contain at least one selectable marker.

According to a preferred embodiment, mamallian CHO wild type cells, preferably CHO dhfr-/- , CHO K1, CHO S, CHO DG44, CHO Easy C cells are transfected by electroporation, more preferably by nucleofection which is an optimized electroporation AMAXA® Nucleofector® Technology (Lonza), with at least two vectors (co-transfection) or with one expression vector in non-adherent culture in a serum-free cell culture medium. In the first case, the different expression vectors are co-transfected either simultaneously or successively into the CHO cell.

According to another preferred embodiment, wild type CHO cells, preferably wild type CHO cells dhfr-/- are transfected by liposome-mediated transfection, using compound like Lipofectamine2000® and the like, with at least two expression vector (co-transfection) or one expression vector in adherent in a 2% serum cell culture medium. In the first case, the different expression vectors are co-transfected either simultaneously or successively into the CHO cell.

The cell line of the present invention is capable of producing all kinds of antibodies that generally comprise equimolar proportions of light and heavy chains.

The invention therefore includes chimeric, humanized or human antibodies.

Also included in the invention are altered antibodies such as hybride antibodies in which the heavy and light chains are homologous to a natural antibody but are combined in a way that would not occur naturally. For example, a bispecific antibody has antigen binding sites specific to more than one antigen. The constant region of the antibody may relate to one or other of the antigen binding regions or may be from a further antibody.

Altered antibodies, such as chimeric antibodies have variable regions from one antibody and constant regions from another. Thus, chimeric antibodies may be species/species chimaeras or class/class chimaeras. Such chimeric antibodies may have one or more further modifications to improve antigen binding ability or to alter effector functioning.

Another form of altered antibody is a humanized, CDR-grafted or SDR-grafted antibody including a composite antibody, wherein parts of the hypervariable regions in addition to the CDRs are transferred to the human framework.

Additional amino acids in the framework or constant regions of such antibodies may be altered. Included in the definition of altered antibody are Fab fragments which are roughly equivalent to the Y branch portions of the heavy and light chains; these may include incomplete fragments or fragments including part of the Fc region.

Thus, within the scope of the invention is included, any altered antibody in which the amino acid sequence is not one which exists in nature.

The human IgG Fc region may be a region of IgG sub-class. It may be an Fc region of IgG1, IgG2, IgG3 or IgG4. In an embodiment, the Fc region is an igG1 Fc region.

Purification of antibodies produced in cell expression systems is known and routinely determined and practiced by those having skill in the art.

The invention provides a protein of interest having a specific profile of glycosylation. As used herein the terms "protein of interest" refers to a monoclonal antibody without being limited thereto.

More specifically, the invention provides an antibody having no or low fucose.

The instant invention further provides a method for producing at least one glycoprotein or antibody of interest in wild type CHO cell, said method comprising the steps of preparing wild type CHO cell according to the invention by transfection or co-transfection one of one or several expression vectors.

Transfection may be performed either on adherent or suspension wild type CHO cells; culturing said transfected wild type CHO cell under suitable conditions and in a cell culture medium; and harvesting the biological product of interest from said transfected wild type CHO cell, the cell culture medium, or both said wild type CHO cell and said medium.

The culturing of said transfected wild type CHO cells can be performed according to the cell culture techniques well-known by the man skilled in the art. As cell cultures that may be used, one may mention, without limitation continuous culture, batch culture and fed-batch culture.

By "passage" it is meant the number of times the cells in the culture, that grow either in suspension or in adherence, have been sub-cultured or passed in a new vessel. This term is not synonymous with population doubling or generation which is the time needed by a cell population to replicate one time; that is to say, roughly the time for each cells of a population to replicate. For example, CHO Easy C or CHO DG44 has a population doubling time (PDT) of around 24 hours.

In a continuous culture, for example, fresh culture medium supplement (i.e. feeding medium) is provided to the cells during the culturing period, while old culture medium is removed daily and the product is harvested, for example, daily or continuously.

In continuous culture, feeding medium can be added daily and can be added continuously, i.e., as a drip or infusion. For continuous culturing, the cells can remain in culture as long as is desired, so long as the cells remain alive and the environmental and culturing conditions are maintained. In batch culture, cells are initially cultured in medium and this medium is either removed, replaced, or supplemented, i.e., cells are not "fed" with new medium, during or before the end of the culturing run. The desired product is harvested at the end of the culturing run.

For fed-batch cultures, the culturing run time is increased by supplementing the culture medium one or more times daily (or continuously) with fresh medium during the run, i.e., the cells are "fed" with new medium ("feeding medium") during the culturing period.

As is appreciated by those having skill in the art, tissue culture dishes, T-flasks and spinner flasks are typically used on a laboratory scale. For culturing on a larger scale (e. g. 3L, 7L, 20L, 100L, 500 L, 5000 L, and the like), procedures including, but not limited to, a fluidized bed bioreactor, a hollow fiber bioreactor, roller bottle culture, or stirred tank bioreactor systems can be used. Microcarriers may or may not be used with the roller bottle or stirred tank bioreactor systems. The systems can be operated in a batch, continuous, or fed-batch mode. In addition, the culture apparatus or system may or may not be equipped with a cell separator using filters, gravity, centrifugal force, and the like.

In the cell culture processes or methods of this invention, the cells can be maintained in a variety of cell culture media, i.e. basal culture media, as conventionally known in the art which can be supplemented with nutrients and the like. such as without limitation an energy source (usually in the form of a carbohydrate such as glucose); all essential amino acids, and generally the twenty basic amino acids, plus cysteine; vitamins and/or other organic compounds typically required at low concentrations; lipids or free fatty acids, e.g., linoleic acid; and trace elements, e.g., inorganic compounds or naturally occurring elements that are typically required at very low concentrations, usually in the micromolar range.

Commercially available media can be utilized and include, for example and whithout limitation, Ham's F 12 Medium (Sigma, St. Louis, MO), Dulbecco's Modified Eagles Medium (DMEM, Sigma), optipro medium (Invitrogen, Carlsbad, CA), CD DG44 medium (Invitrogen), CHTS medium (Cell Culture Technologies, Gravesano, Switzerland), CHP1 medium (Cell Culture Technologies), CHP5 medium (Cell Culture Technologies), CHP6 medium (Cell Culture Technologies), Select CD1000 medium (Becton Dickinson, Sparks, MD), CDM4CHO medium (Hyclone, Southlogan, Utah) or Excell media (SAFC, Lenexa, KS).

To the foregoing exemplary media can be added supplementary components or ingredients, including optional components, in appropriate concentrations or amounts, as necessary or desired, and as would be known and practiced by those having in the art using routine skill. In addition, cell culture conditions suitable for the methods of the present invention are those that are typically employed and known for batch, fed-batch, or continuous culturing of cells, with attention paid to pH, e.g. about 6.5 to about 7.5; dissolved oxygen (O₂), e.g., between about 5-90% of air saturation and carbon dioxide (CO₂), agitation and humidity, in addition to temperature.

Once harvest the biological product of interest is usually concentrated. Once obtained in concentrated form, any standard technique, such as preparative disc gel electrophoresis, ion-exchange chromatography, gel filtration, size separation chromatography, isoelectric focusing and the like may be used to purify, isolate, and/or to identify the heterologous protein. Those skilled in the art may also readily devise affinity chromatographic means of heterologous protein purification, especially for those instances in which a binding partner of the heterologous protein is known, for example, antibodies. Isolation of monoclonal antibodies is simplified and safety is enhanced due to the absence of additional human or animal proteins in the culture. The absence of serum further increases reliability of the system since use of synthetic media, as contemplated herein, enhances reproducibility.

The present invention provides a method for the production of an antibody which comprises culturing a transfected wild type CHO cell of the present invention. Culture of the wild type CHO cells may be carried out in serum-containing or preferably serum and protein free media. The resulting antibody may be purified and formulated in accordance with standard procedures.

Expression of both chains of MAbs in substantially equimolar proportions enables optimum yields of functional antibody to be obtained. The transfected wild type CHO cells of the invention, and more specifically CHO DG44 or CHO Easy C cells, are able to produce at least approximately 10 pg/cell/day of immunoglobulin after cell transfection and cell sorter, 10 pg/cell/day of immunoglobulin in batch culture, preferably at least 15 pg/cell/day of immunoglobulin in batch culture, more preferably at least 25 pg/cell/day of immunoglobulin in batch culture, even more preferably at least 35 pg/cell/day of immunoglobulin in batch culture or higher following bioprocess optimization. The two chains assemble within the cell and are then secreted into the culture medium as functional antibody.

In an embodiment, antibodies are concerned. Antibodies may include, but are not limited to monoclonal antibodies (MAbs), humanized or chimeric antibodies, camelized antibodies, single chain antibodies (scFvs), Fab fragments, F(ab')₂ fragments, disulfide-linked Fvs (sdFv) fragments, anti-idiotypic (anti-Id) antibodies, intra-bodies, synthetic antibodies, and epitope-binding fragments of any of the above. The term "antibody" also refers to fusion protein that includes a region equivalent to the Fc region of an immunoglobulin.

Examples of antibodies without antibody specificity limitation within the scope of the present invention include those comprising the amino acid sequences of the following antibodies, with a modification in the Fc region in accordance with the invention: anti-HER2 antibodies, anti-CD20 antibodies anti-CD52 antibodies.

The invention also relates to an optimized antibody or an antibody population, produced in wild type CHO cell, and having only an increased ADCC activity without CDC activity compared to the wild type murine parental antibody produced in hydridoma or to the wild type chimeric parental antibody wild-type produced in CHO cell line, preferably CHO dhrf-/-, CHO- K1, CHO-DG44, CHO-S, CHO-Easy C.

The invention also relates to an optimized antibody or an antibody population, produced in wild type CHO cell, and having both an increased ADCC activity and an induced CDC activity compared to the wild type murine parental antibody produced in hydridoma or to the wild type chimeric parental antibody wild-type produced in CHO cell line, preferably CHO dhrf-/-, CHO- K1, CHO-DG44, CHO-S, CHO-Easy C.

As used herein, the term increased Fc-mediated cellular cytotoxicity is defined as either an increase in the number of "antibody-targeted cells" that are lysed in a given time, at a given concentration of antibody, or of Fc-fusion protein, in the medium surrounding the target cells, by the mechanism of Fc-mediated cellular cytotoxicity defined above, and/or a reduction in the concentration of antibody, in the medium surrounding the target cells, required to achieve the lysis of a given number of "antibody-targeted cells", in a given time, by the mechanism of Fc-mediated cellular cytotoxicity.

The increase in Fc-mediated cellular cytotoxicity is relative to the cellular cytotoxicity mediated by the parental wild type chimeric antibody without Fc mutation produced by the same type of host cells, using the same standard production, purification, formulation and storage methods, which are known to those skilled in the art.

The antibody is preferably selected among IgG, and more preferably among IgG1, IgG2, IgG3 and IgG4. More preferably the antibody is an IgG1.

The wild type CHO cells protein production platform of Fc engineered MAbs is therefore useful to increase Fc-mediated cellular cytotoxicity against undesirable cells mediated by an immunoglobulin or a fragment thereof or by any biological molecule carrying a Fc region of an immunoglobulin region, or an equivalent to the Fc region of an immunoglobulin.

The invention provides that the transfected wild type CHO cells of the invention allow expressing a large proportion of Fc variant antibodies characterized as having substantially reduced content of fucose as compared to the native parental chimeric MAb produced using CHO cell line.

Among a recombinant antibody population produced in wild type CHO cells, said antibody is characterized as having approximately 20 % or more of non-fucosylated N-linked oligosaccharides structures G0, G1 and G2.

Among a recombinant antibody population produced in wild type CHO cells, said antibody is characterized as having approximately specific non-fucosylated N-linked oligosaccharides structures G0, G1 and G2 related to the Fc variant.

Antibody glycosylated by the wild type CHO cells maintain antigen binding capability and effector functionality.

Interestingly, the inventors have now demonstrated that the antibody produced by the method of the invention have increased Fc-mediated cellular toxicity.

For example, the variant Fc7, Fc20, 24 or 34 antibody produced in the wild type CHO cells have an increased ADCC activity compared to the wild type chR005-1 Fc0 antibody produced in the same CHO cells. This is achieved by providing the variant antibodies of interest with specific and restricted wild type CHO glycosylation pattern. Moreover, Fc24 and Fc34 variant antibody have a CDC activity, whereas Fc7 or Fc20 has not.

The instant invention relates to the antibodies according the invention as a medicament.

The higher cytotoxicity activity of wild type CHO produced Fc optimised IgG will allow to reduced the amount of antibody administrated to patients and to decreased treatment associated costs.

In addition, the present invention allows the person skilled in the art to produce antibodies having various glycosylation profiles, including low fucose level as defined herein, these profiles resulting from the Fc mutations and the production in the host cells according to the invention.

The invention therefore provides the use of these antibodies in the manufacture of a medicament for the prophylactic and therapeutic treatment of disorders against which the antibody is designed.

Also provided is a method of treating a human being having such a disorder comprising administering to said individual a prophylactic or therapeutically effective amount such an antibody.

The invention also covers the use of a antibody according to the invention for the preparation of a pharmaceutical composition for the prevention or the treatment of human and animal diseases. Such pharmaceutical compositions preferably include, in addition to the monoclonal antibody, a physiologically acceptable diluent or carrier possibly in a mixture with other agents such as for example an antibiotic.

Suitable carriers include but are not limited to physiological saline, phosphate buffered saline, phosphate buffered saline glucose and buffered saline. Alternatively, the biological product such as an antibody may be lyophilised (freeze dried) and reconstituted for use when needed by the addition of an aqueous buffered solution as described above.

Therefore, the invention provides a pharmaceutical composition comprising the biological product of the invention and a pharmaceutical acceptable carrier. The dosages of such biological products will vary with the condition being treated and the recipient of the treatment.

BRIEF DESCRIPTION OF THE DRAWINGS

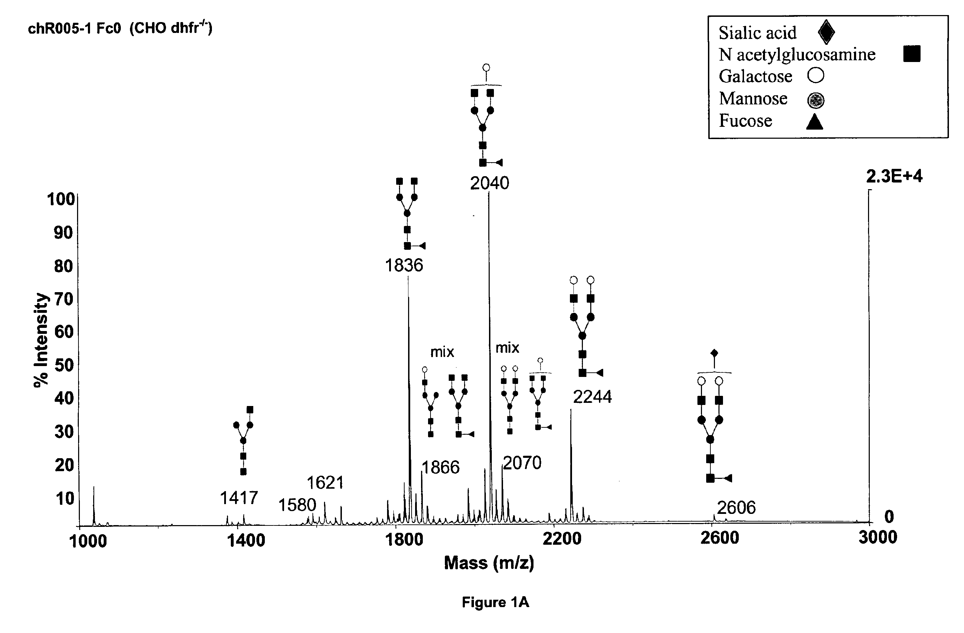

Figure 1A - 1C: N-glycosylation profile analyzed by MALDI-TOF mass spectrum of chR005-1 Fc0 produced from different wild type CHO cells. Mass spectrum analysis of N-linked glycans attached to the CH2 domain of an IgGI heavy chain at residue Asn 297 produced in different types of CHO cells. Antibody Fc N glycans released by PNGase F digestion were permethyled and analyzed using a MALDI-TOF MS in the positive ion mode using a DHB matrix. Representative experiment of (A) CHO dhfr-/- (n=1), (B), CHO DG44 (n=1), and (C) CHO Easy (n=3).

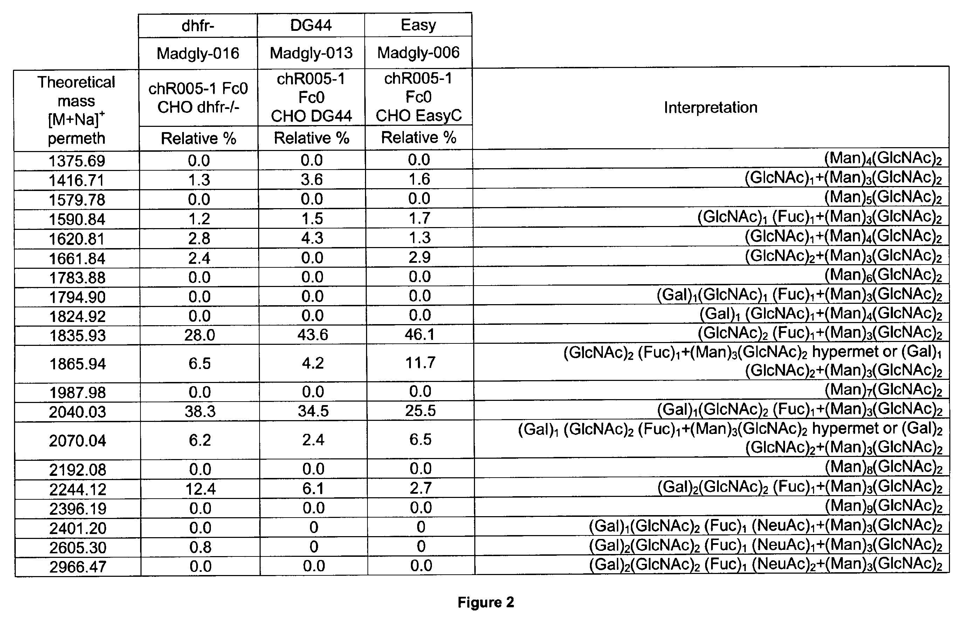

Figure 2: IgG N-linked glycans in the native chR005-1 Fc0 produced from different type CHO cells. Molecular mass are permethylated glycans, detected as [M+Na]+ by Maldi mass spectrometry. The MAb was produced in different types of CHO cells. Representative experiment of (A) CHO dhfr-/- (n=1), (B), CHO DG44 (n=1), and (C) CHO Easy (n=1)

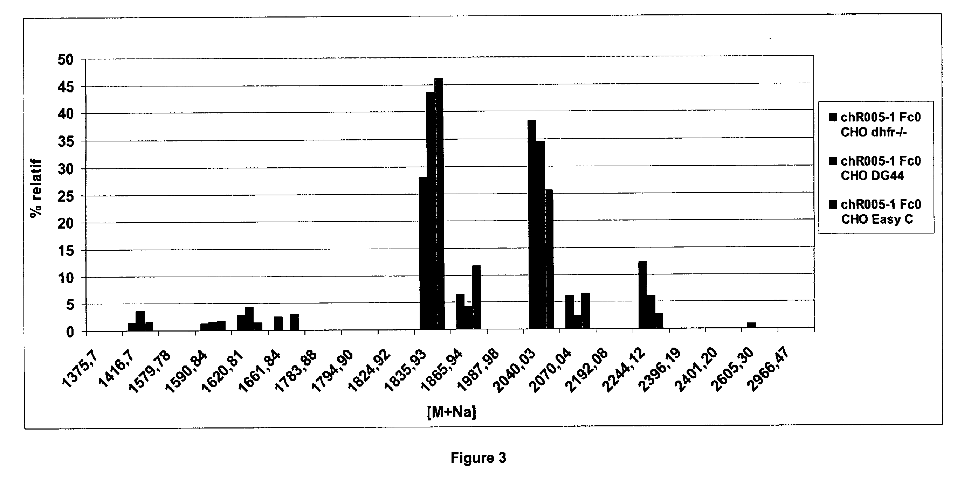

Figure 3: Comparison of glycosylation profiles in the native chR005-1 Fc0 antibody produced from different type CHO cells. Most of the native chR005-1 Fc0 antibodies produced in this CHO cell line panel had a common N-linked oligosaccharide structure of a bi-antennary type that comprised long chains with terminal GIcNac, that were galactosylated. The three glycoforms GOF, G1 F and G2F were founded on MAbs produced on both CHO cell lines. Representative experiment of CHO dhfr-/- (n=1), CHO DG44 (n=1), and CHO Easy (n=3).

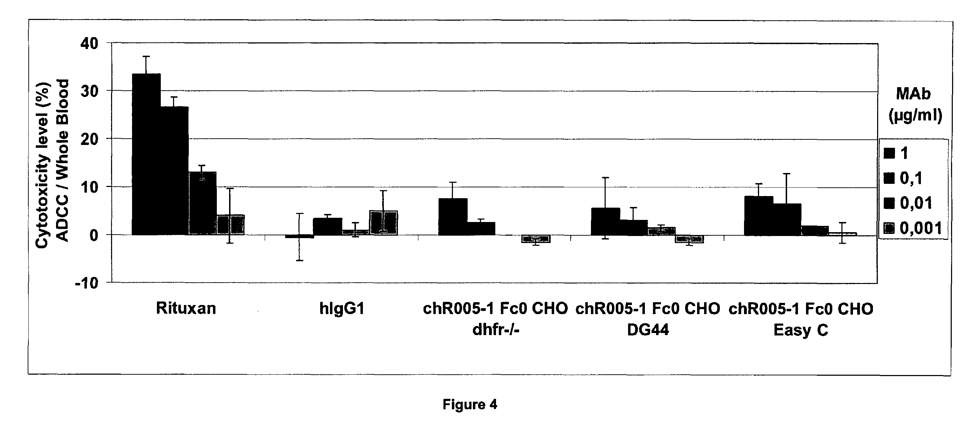

Figure 4: Whatever the type of CHO cells, the native chR005-1 Fc0 did not trigger ADCC activity on Burkitt's lymphoma cells. Calcein-AM loaded Raji cells (1 x10⁵) were incubated with interest MAbs for 20 min at 4°C. The effector cells (50µl/ well of human whole blood) were added for 4 hours at 37°C under shaking condition. After centrifugation, supernatants were harvested and calcein-AM fluorescence was measured on fluorometer. Lysis level was calculated following the formula: (experimental release - (target + effector spontaneous realease) / (maximal release - target spontaneous release)*100. Maximal release value was obtained by treating target cells with Triton X-100. Mean +/- SD of 2independent experiments.

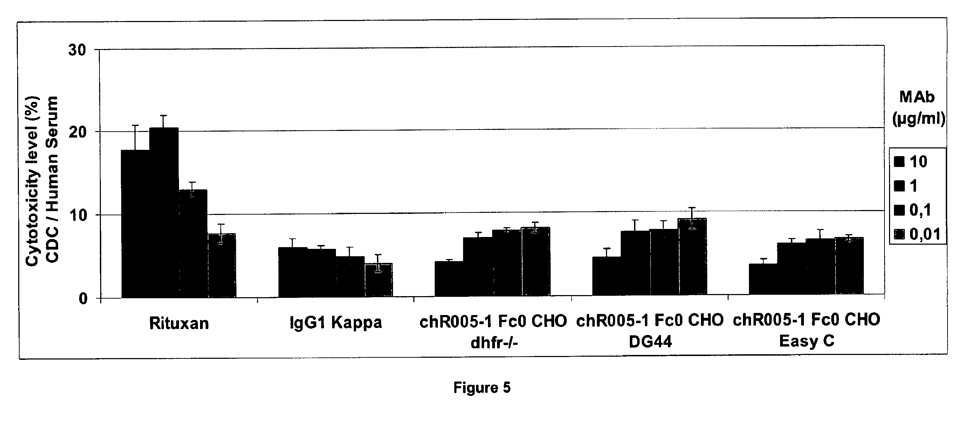

Figure 5: Whatever the type of CHO cells, the native chR005-1 Fc0 did not trigger CDC activity on Burkitt's lymphoma cells. The target Raji cells (5x10⁴) were incubated with interest MAbs for 20 min at 4°C. Then 5 µl of natural human complement was added for 4 hours at 37°C under shaking condition. After incubation, supernatants were harvested and lactate deshydrogenase (LDH) was measured on fluorometer. Lysis level was calculated following the formula: (experimental release - target spontaneous release) / (maximal release - target spontaneous release)*100, where target without natural complement represented spontaneous release. Maximal release value was obtained by treating target cells with Triton X-1 00. Mean +/- SD of one independent experiment.

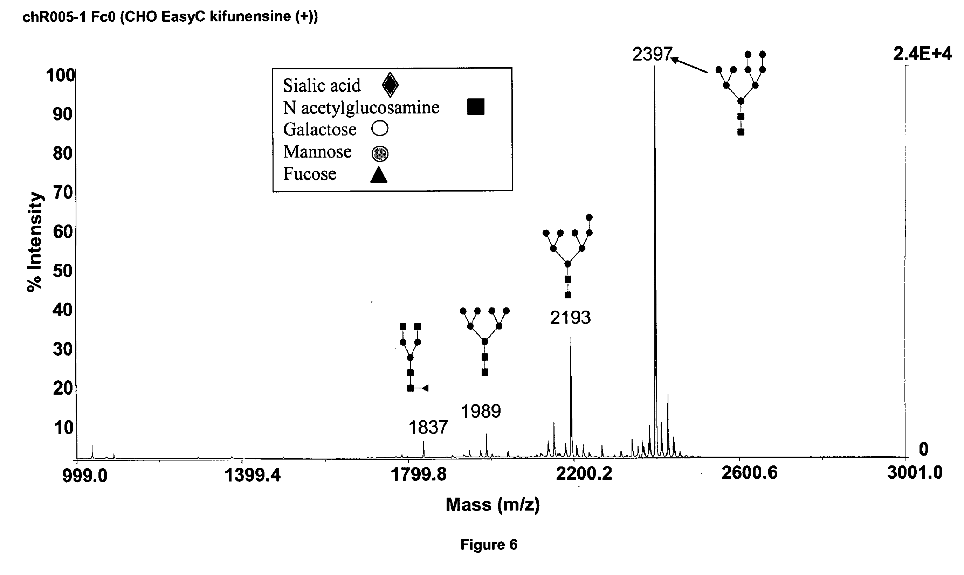

Figure 6: N-glycosylation profile analyzed by MALDI-TOF mass spectrum of chR005-1 Fc0 produced from the wild type CHO Easy C cells in the presence of kifunensine. Mass spectrum analysis of N-linked glycans attached to the CH2 domain of an IgGI heavy chain at residue Asn 297. Antibody Fc N glycans released by PNGase F digestion were permethyled and analyzed using a MALDI-TOF MS in the positive ion mode using a DHB matrix. Data represent one independent experiment.

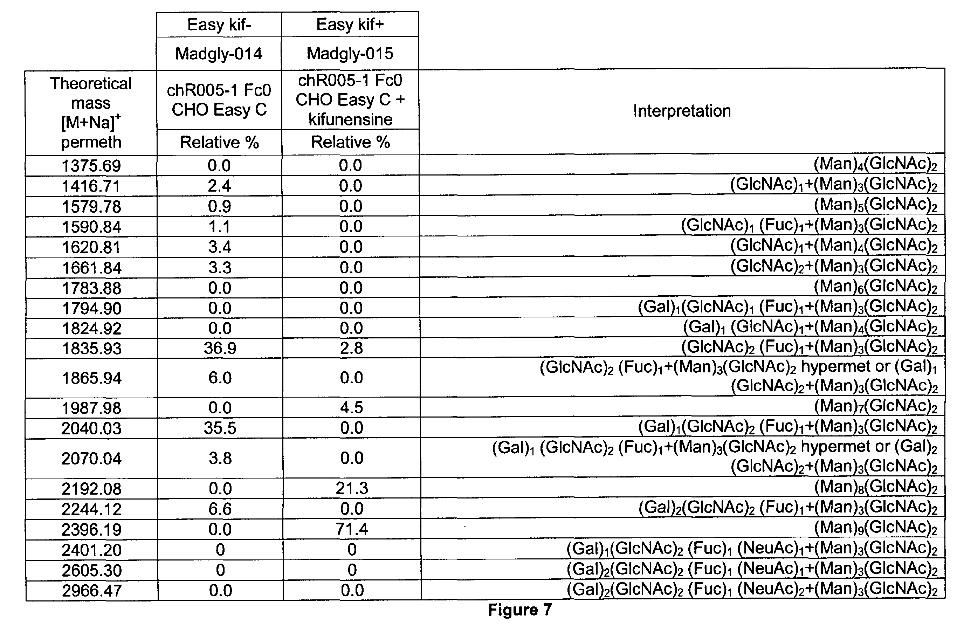

Figure 7: IgG N-linked glycans in the native chR005-1 Fc0 produced from the wild type CHO Easy C cells in the presence of kifunensine. Molecular mass are permethylated glycans, detected as [M+Na]+ by Maldi mass spectrometry. The MAb was produced in the CHO Easy cells. Representative experiment.

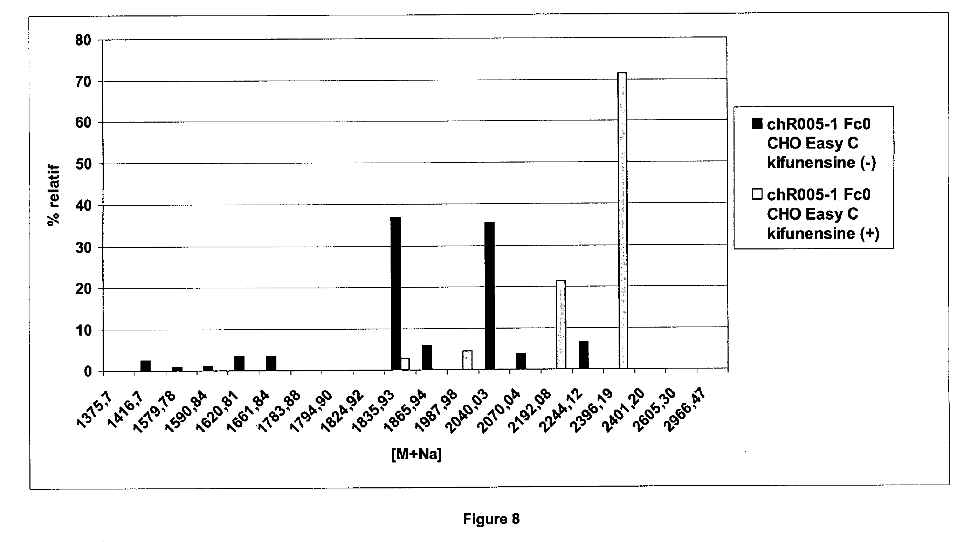

Figure 8: Comparison of glycosylation profiles in the native chR005-1 Fc0 following the kifunensine treatment. Different glycosylation profiles in the native chR005-1 Fc0 antibody were observed with or without kifunensine. The MAb was produced in the CHO Easy cells. Representative experiment.

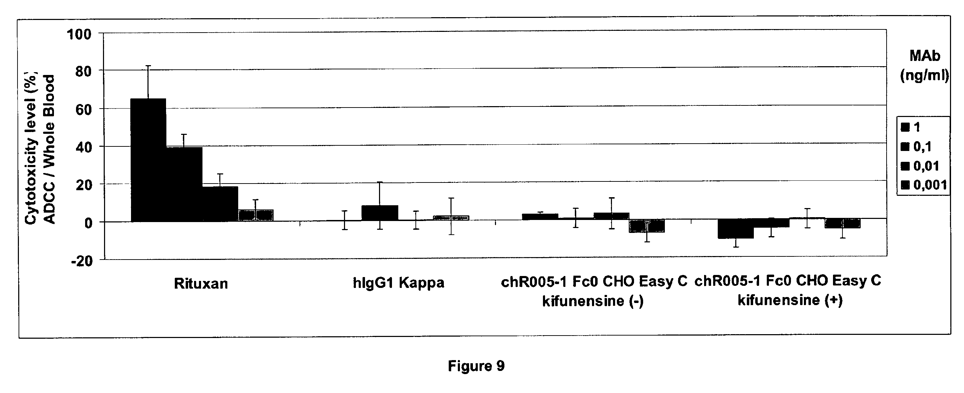

Figure 9: The kifunensine induced non fucosylated MAb chR005-1 Fc0 did not trigger ADCC activity on Burkitt's lymphoma cells. Calcein-AM loaded Raji cells (1x10⁵) were incubated with interest MAbs for 20 min at 4°C. The effector cells (50 µl per well of whole Blood) were added for 4 hours at 37°C under shaking condition. After centrifugation, supernatants were harvested and calcein-AM fluorescence was measured on fluorometer. Lysis level was calculated following the formula: (experimental release - (target + effector spontaneous realease) / (maximal release - target spontaneous release)*100. Maximal release value was obtained by treating target cells with Triton X-100. Data represent one independent experiment.

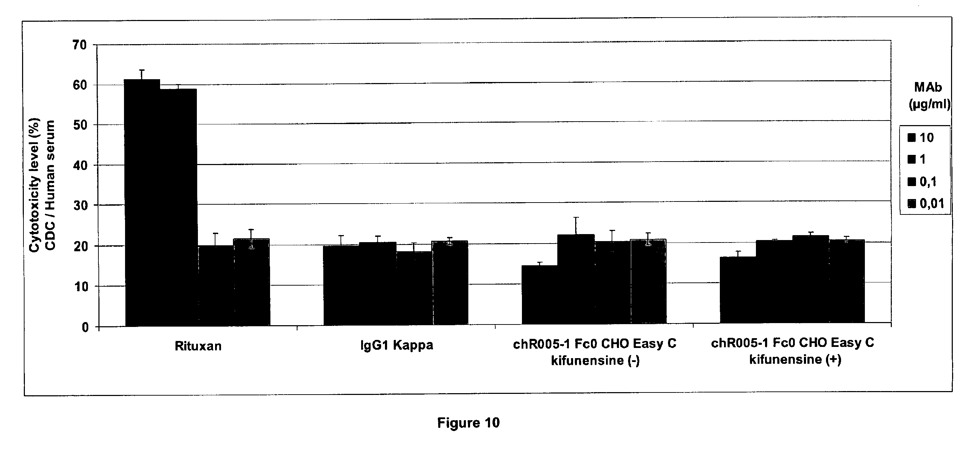

Figure 10: The kifunensine induced non fucosylated MAb chR005-1 Fc0 did not trigger CDC activity on Burkitt's lymphoma cells. The target Raji cells (5x10⁴) were incubated with interest MAbs for 20 min at 4°C. Then 5 µl of natural human complement was added for 4 hours at 37°C under shaking condition. After incubation, supernatants were harvested and lactate deshydrogenase (LDH) was measured on fluorometer. Lysis level was calculated following the formula: (experimental release - target spontaneous release) / (maximal release - target spontaneous release)*100, where target without natural complement represented spontaneous release. Maximal release value was obtained by treating target cells with Triton X-1 00. Data represent one independent experiment.

Figure 11A - 11D: The amino acid and nucleic acid sequences of chR005-1 Fc variant MAbs. Amino acids are shown as one-letter codes. According to the literature, the amino acid numbering of Fc region is based to the Kabat data base, (CH1: aa n°118 to 215; Hinge: aa n °216 to 230; CH2: aa n °231 to 340; CH3: aa n °341 to 447). chR005-1 Fc7 : SEQ ID NO: 1 for amino acid sequence, SEQ ID NO: 2 for nucleic acid sequence; chR005-1 Fc20 : SEQ ID NO: 3 for amino acid sequence, SEQ ID NO: 4 for nucleic acid sequence; chR005-1 Fc24 : SEQ ID NO: 5 for amino acid sequence, SEQ ID NO: 6 for nucleic acid sequence; chR005-1 Fc20 : SEQ ID NO: 7 for amino acid sequence, SEQ ID NO: 8 for nucleic acid sequence.

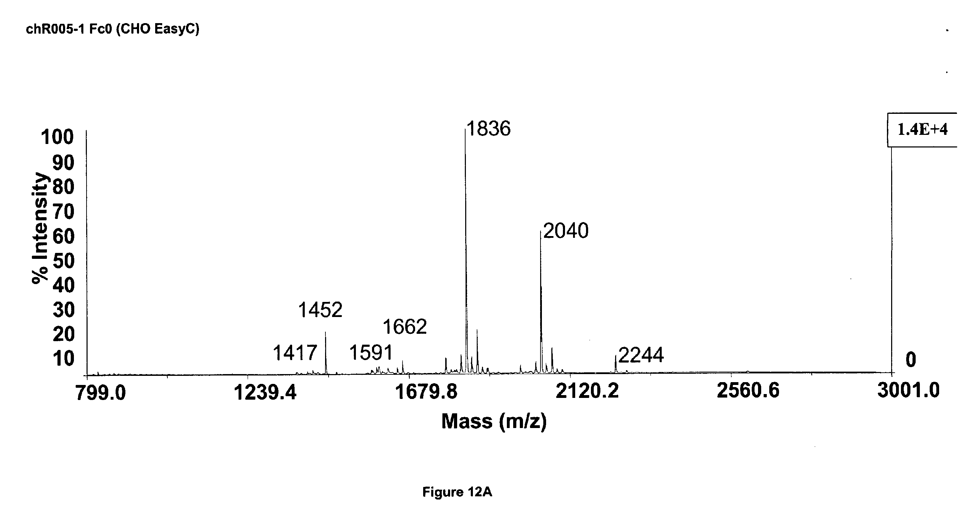

Figure 12A - 12D: N-glycosylation profile analyzed by MALDI-TOF mass spectrum of chR005-1 Fc variant antibodies produced from the wild type CHO Easy C cells. Mass spectrum analysis of N-linked glycans attached to the CH2 domain of an IgGI heavy chain at residue Asn 297 produced in CHO Easy C cells. Antibody Fc N glycans released by PNGase F digestion were permethyled and analyzed using a MALDI-TOF MS in the positive ion mode using a DHB matrix. Representative experiment of (A) chR005-1 Fc0 (n=3), (B) chR005-1 Fc7 (n=2), (C) chR005-1 Fc20 (n=2) and (D) chR005-1 Fc24 (n=1).

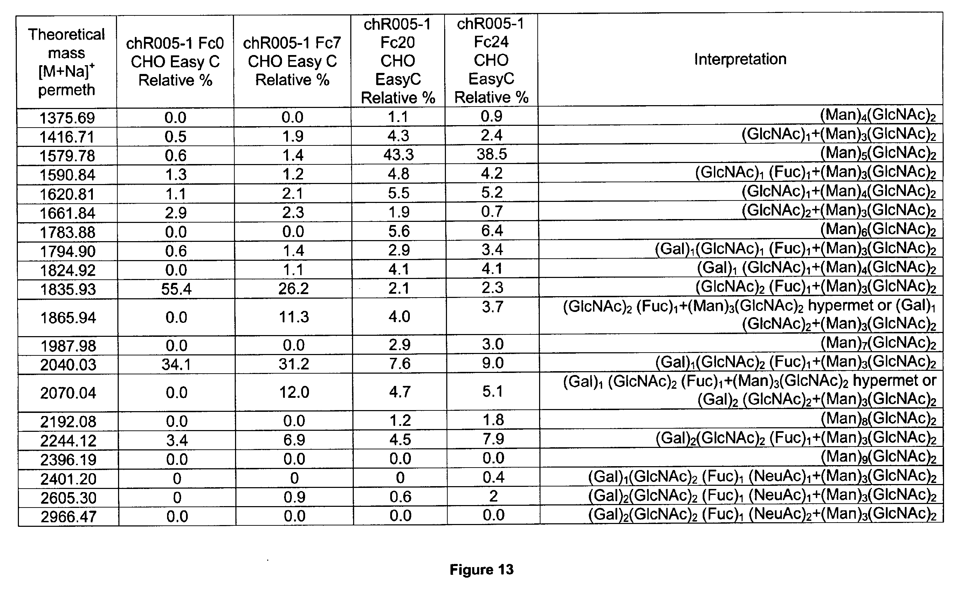

Figure 13: IgG N-linked glycans of chR005-1 Fc variant antibodies produced from the wild type CHO Easy C cells. Molecular mass are permethylated glycans, detected as [M+Na]+ by Maldi mass spectrometry. The MAb panel was produced from CHO Easy C cells. Different glycosylation profiles among the Fc variant antibody panel were observed. Representative experiment of chR005-1 Fc0 (n=3), chR005-1 Fc7 (n=2), chR005-1 Fc20 (n=2) and chR005-1 Fc24 (n=1).

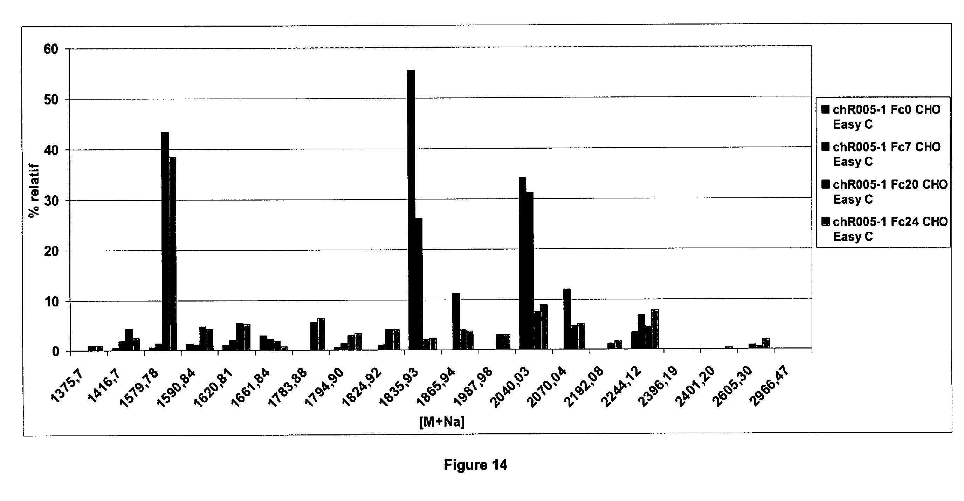

Figure 14: Comparison of glycosylation profiles of chR005-1 Fc variant antibodies produced from the wild type CHO Easy C cells. Representative experiment of chR005-1 Fc0 (n=3), chR005-1 Fc7 (n=2), chR005-1 Fc20 (n=2) and chR005-1 Fc24 (n=1).

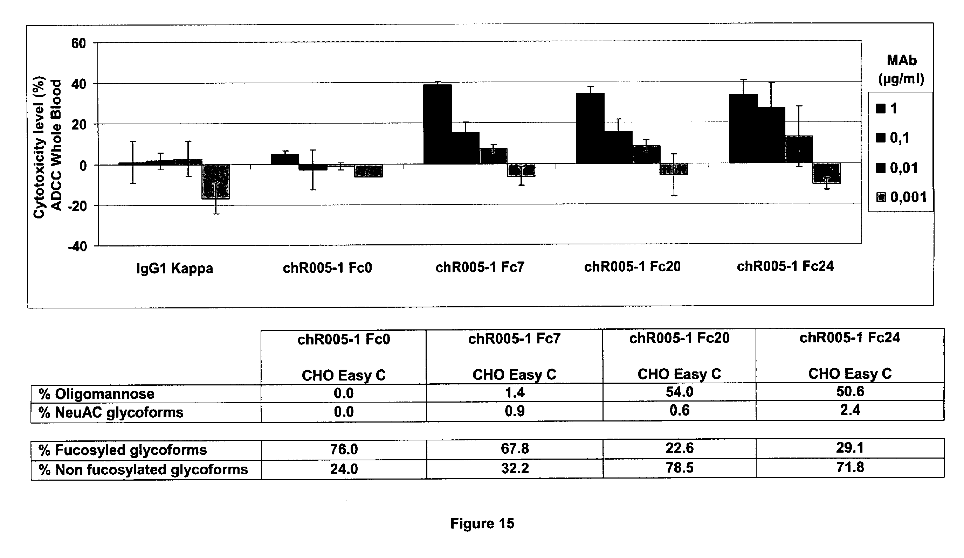

Figure 15: Influence of glycosylation profiles on ADCC potency of Fc variant antibody produced from CHO Easy C cells. Calcein-AM loaded Raji cells (1x10⁵) were incubated with interest MAbs for 20 min at 4°C. The effector cells (50 µl of human whole blood) were added for 4 hours at 37°C under shaking condition. After centrifugation, supernatants were harvested and calcein-AM fluorescence was measured on fluorometer. Lysis level was calculated following the formula: (experimental release - (target + effector spontaneous realease) / (maximal release - target spontaneous release)*100. Maximal release value was obtained by treating target cells with Triton X-100. Data represent one independent experiment.

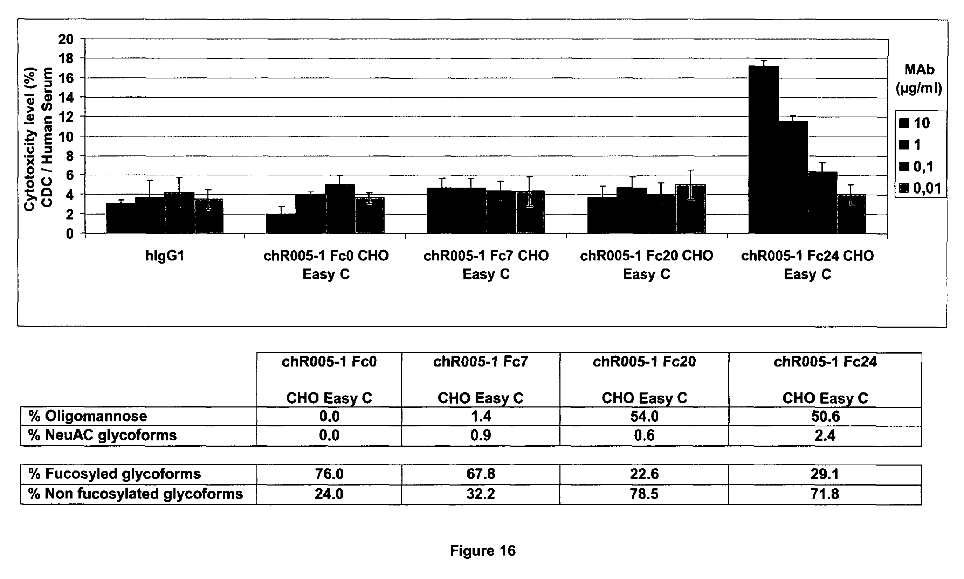

Figure 16: No influence of glycosylation profiles on CDC potency of Fc variant antibody produced from CHO Easy C cells. The target Raji cells (5x10⁴) were incubated with interest MAbs for 20 min at 4°C. Then 5 µl of natural human complement was added for 4 hours at 37°C under shaking condition. After incubation, supernatants were harvested and lactate deshydrogenase (LDH) was measured on fluorometer. Lysis level was calculated following the formula: (experimental release - target spontaneous release) / (maximal release - target spontaneous release)*100, where target without natural complement represented spontaneous release. Maximal release value was obtained by treating target cells with Triton X-1 00. Mean +/- SD of three independent experiments.

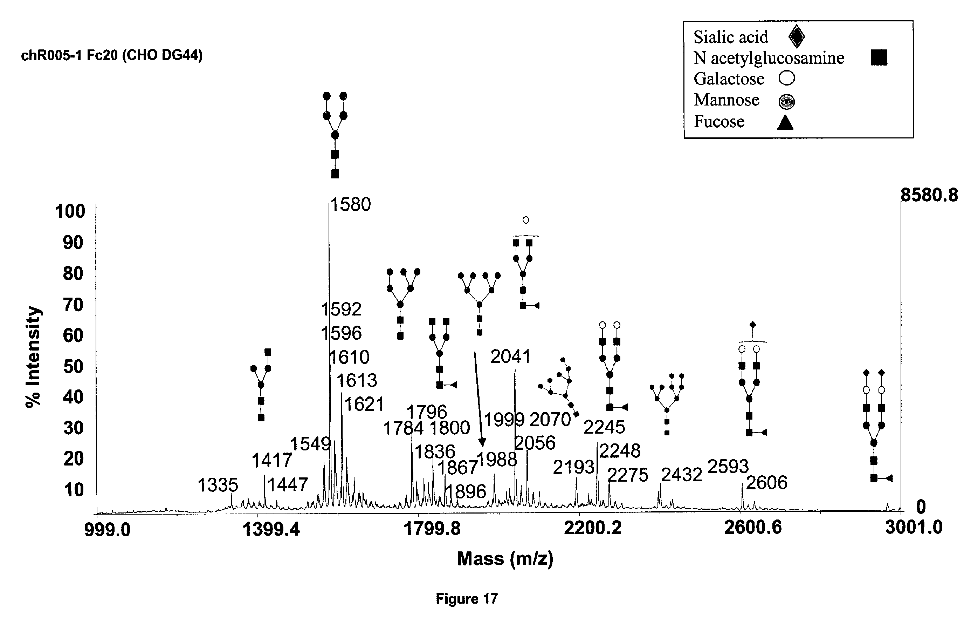

Figure 17: N-glycosylation profile analyzed by MALDI-TOF mass spectrum of chR005-1 Fc20 produced from the wild type CHO DG44 cells. Mass spectrum analysis of N-linked glycans attached to the CH2 domain of an IgGI heavy chain at residue Asn 297 produced in CHO DG44 cells. Antibody Fc N glycans released by PNGase F digestion were permethyled and analyzed using a MALDI-TOF MS in the positive ion mode using a DHB matrix. Representative experiment.

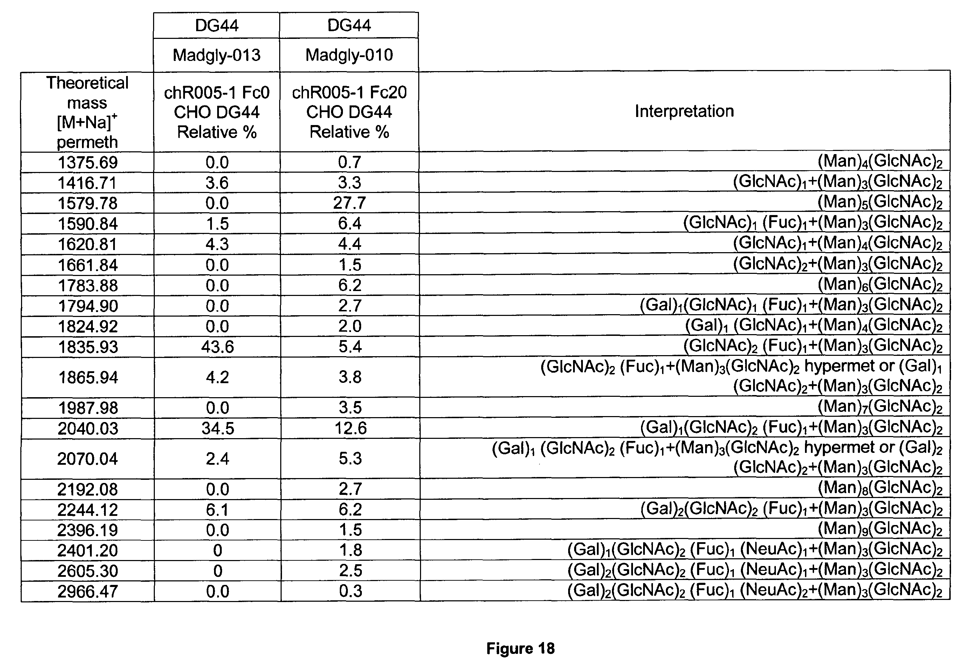

Figure 18: IgG N-linked glycans in the chR005-1 variant Fc20 compared to the native chR005-1 Fc0 from the wild type CHO DG44 cells. Molecular mass are permethylated glycans, detected as [M+Na]+ by Maldi mass spectrometry. Representative experiment.

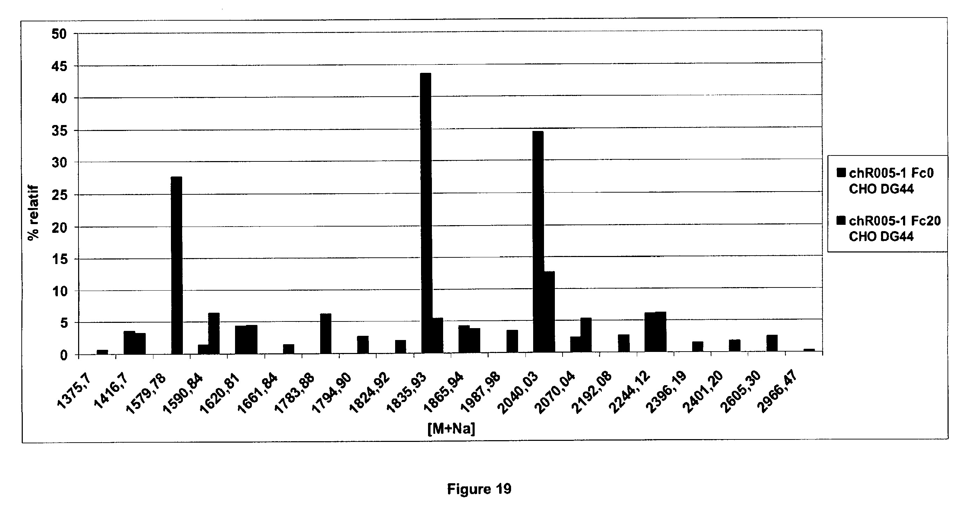

Figure 19: Comparison of glycosylation profiles in the chR005-1 variant Fc20 compared to the native chR005-1 Fc0 from the wild type CHO DG44 cells. Different glycosylation profiles were observed. Representative experiment.

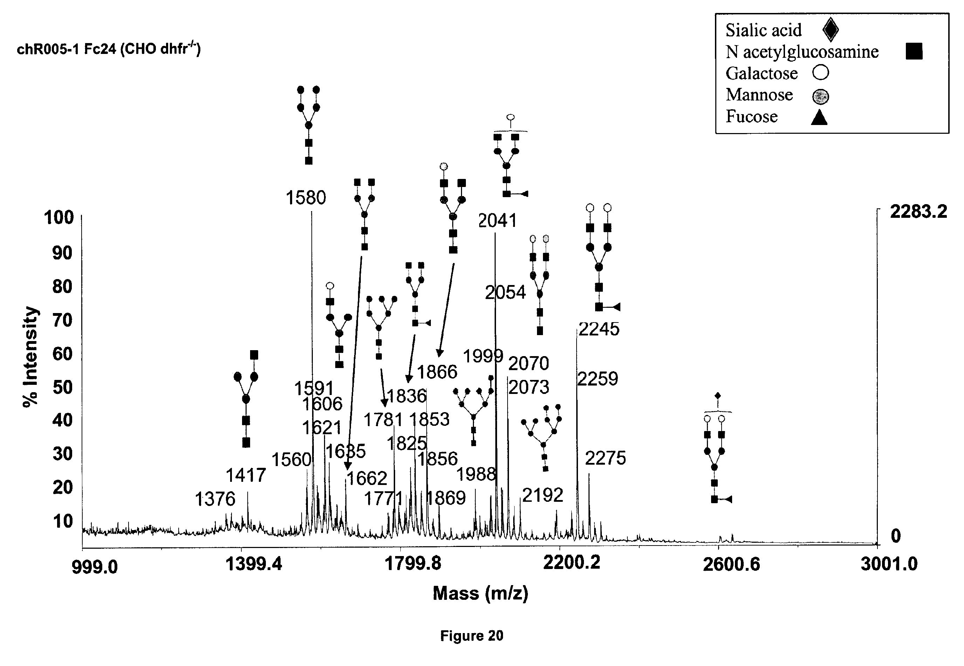

Figure 20: N-glycosylation profile analyzed by MALDI-TOF mass spectrum of chR005-1 Fc24 produced from the wild type CHO dhfr-/- cells. Mass spectrum analysis of N-linked glycans attached to the CH2 domain of an IgGI heavy chain at residue Asn 297 produced in CHO dhfr-/- cells. Antibody Fc N glycans released by PNGase F digestion were permethyled and analyzed using a MALDI-TOF MS in the positive ion mode using a DHB matrix. Representative experiment.

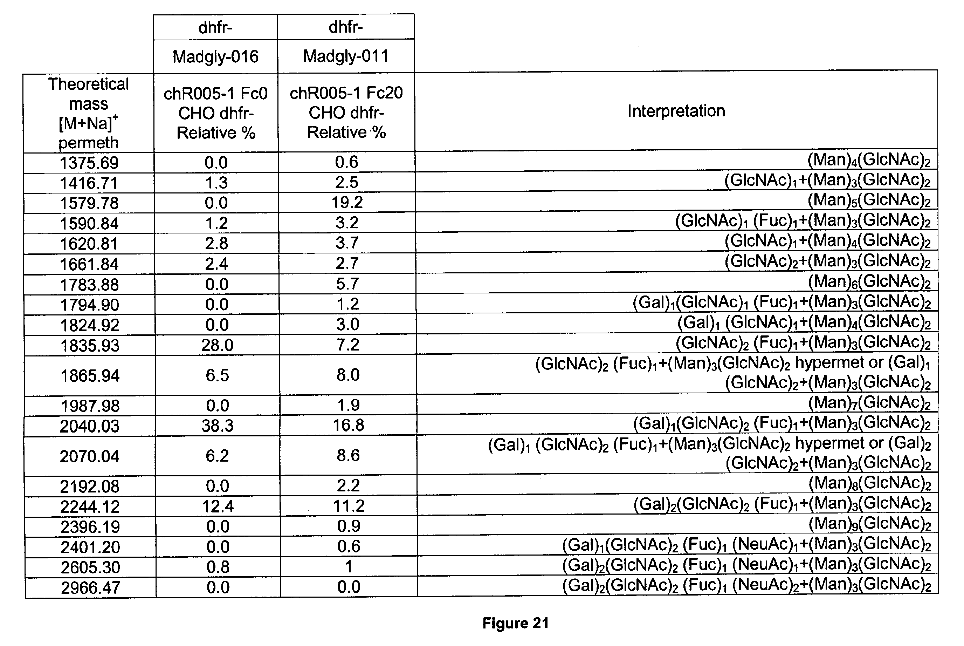

Figure 21: IgG N-linked glycans in the chR005-1 variant Fc24 compared to the native chR005-1 Fc0 from the wild type CHO dhfr-/- cells. Molecular mass are permethylated glycans, detected as [M+Na]+ by Maldi mass spectrometry. Representative experiment

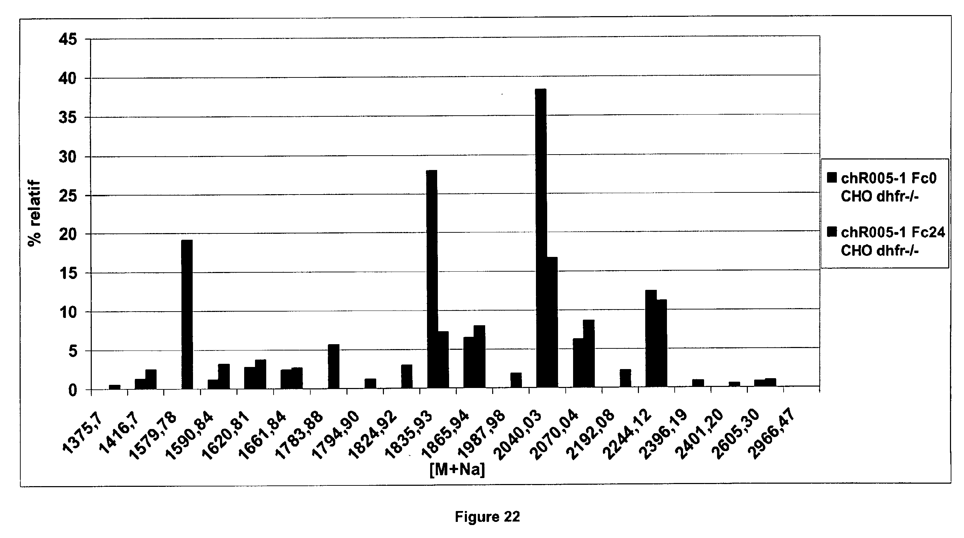

Figure 22: Comparison of glycosylation profiles in the chR005-1 variant Fc24 compared to the native chR005-1 Fc0 from the wild type CHO dhfr-/- cells. Different glycosylation profiles were observed. Representative experiment.

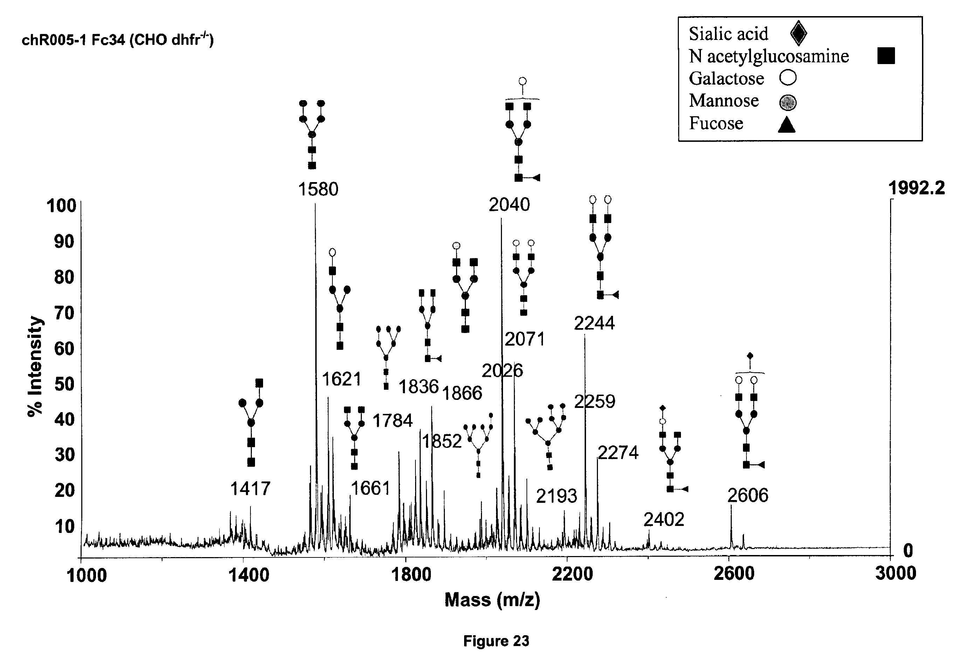

Figure 23: N-glycosylation profile analyzed by MALDI-TOF mass spectrum of chR005-1 F34 produced from the wild type CHO dhfr-/- cells. Mass spectrum analysis of N-linked glycans attached to the CH2 domain of an IgGI heavy chain at residue Asn 297 produced in CHO dhfr-/- cells. Antibody Fc N glycans released by PNGase F digestion were permethyled and analyzed using a MALDI-TOF MS in the positive ion mode using a DHB matrix. Representative experiment.

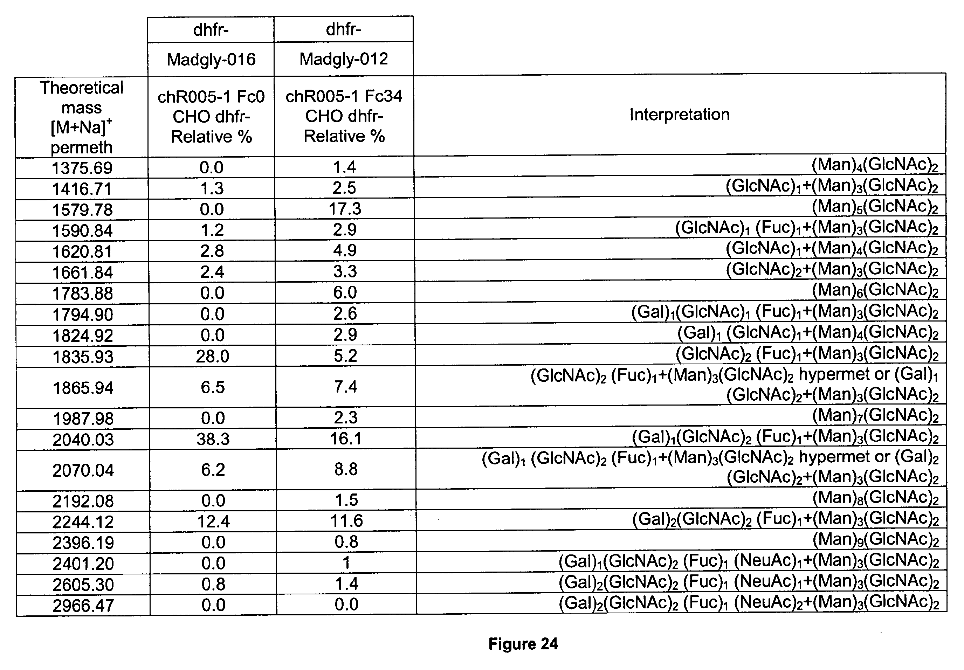

Figure 24: IgG N-linked glycans in the chR005-1 variant Fc34 compared to the native chR005-1 Fc0 from the wild type CHO dhfr-/- cells. Molecular mass are permethylated glycans, detected as [M+Na]+ by Maldi mass spectrometry. Representative experiment.

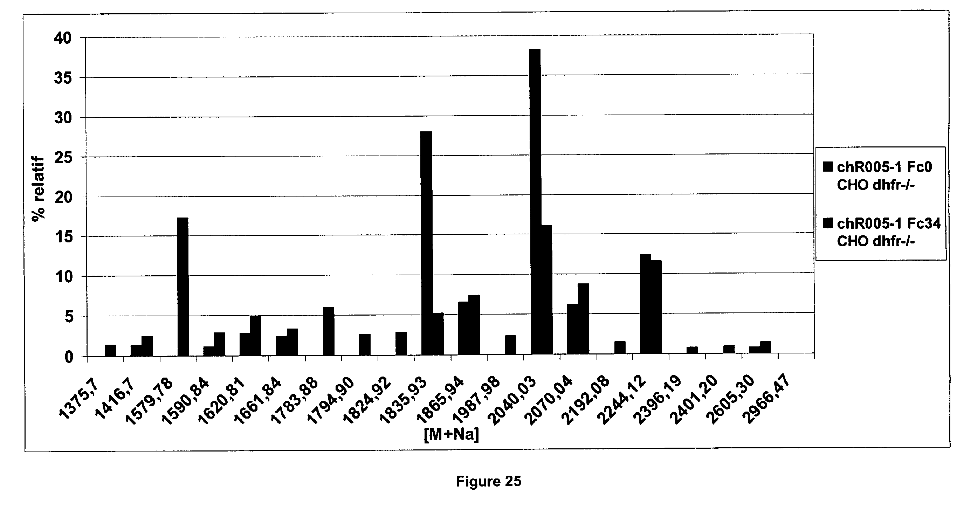

Figure 25: Comparison of glycosylation profiles in the chR005-1 variant Fc34 compared to the native chR005-1 Fc0 from the wild type CHO dhfr-/- cells. Different glycosylation profiles among the Fc variant antibody panel were observed. Representative experiment.

**MATERIALS AND METHODS**

Cells: The CHO dhfr-/-, CHO DG44 or CHO Easy C cell lines were purchased from ATCC (American Type Culture Collection, USA), ECACC (European Collection of Cell culture) or by CCT (Cell culture Technologies).

The Burkitt's lymphoma Raji cell line was obtained from ECACC (European Collection of Cell Culture, UK). Human PBMNCs were purified from leukapheresis of anonymous healthy volunteer donors (Blood Center) using Ficoll-Histopaque density gradient (Sigma). All B-CLL patients enrolled in this study had been defined immunophenotypically as outlined by criteria from the National Cancer Institute Working Group in 1996 (Cheson *et al.,* 1996). Blood was obtained from patients after written informed consent in accordance with the Declaration of Helsinki.

Reagents and antibodies: The IgG1 chimeric negative control was purchassed by Sigma (Saint Quentin Fallavier, France). The wild type chR005-1 Fc0 (also called native IgG1) and all the modified antibodies were generated and produced by iDD biotech (Dardilly, France). The kifunensine was purchased by Sigma (Saint Quentin Fallavier, France)

Construction of antibody variants: Substitutions in the Fc domain were introduced using "megaprimer" method of site-directed mutagenesis (Sarkar *et al.,* 1990). Positions are numbered according to the Kabat® index (Identical V region amino acid sequences and segments of sequences in antibodies of different specificities). Relative contributions of VH and VL genes, minigenes, and complementarity-determining regions to binding of antibody-combining sites were analyzed (Kabat *et al.,* 1991). Heavy and light chain constructs were co-transfected into CHO cells. Antibodies were purified using protein A affinity chromatography (GE Healthcare).

Glycosylation analysis: Release of N-glycans & permethylation were carried out following standard procedures (Ciucanu *et al.,* 1984). IgG were denatured in 0.5% sodium dodecyl sulfate (SDS) and 1% β-mercaptoethanol (+90 °C, 5 min) and deglycosylated by enzymatic digestion 15 hours with PNGase F (ROCHE) at +37°C in phosphate buffer, pH 7.5 (Morelle *et al.,* 2007). N-glycans were purified on Ultra Clean SPE Carbograph (ALLTECH). After elution with 25 % acetonitrile, 0.1 % TFA, the N-glycans were lyophilisated before permethylation. Permethylation using sodium hydroxide procedure (Ciucanu *et al.,* 1984) was performed. After derivatization, the reaction products were purified on C18 Sep Pak Plus (WATERS) and lyophilisated before MALDI TOF MS. Analysis of protein glycosylation was determined by mass spectrometry (Morelle *et al.,* 2007). The purified permethylated N-glycans were solubilised with 20 µl of 50% methanol. 1 µl was mixed with 1µl of 2,5 DHB (LaserBiolabs) matrix solution (10 mg /ml, 50% methanol). Positive ion reflectron MALDI mass spectra were acquired on a MALDI TOF MS VOYAGER DE PRO (Applied Biosystems). The spectra were obtained by accumulation of 500 shots and were calibrated with an external standard (LaserBiolabs). The acceleration and reflector voltage conditions were set as follows: voltage 20kV, grid 75%, guide wire 0.002% and delay time 175 ns. MALDI-PSD fragmentation was performed on selected ions using the following conditions: target voltage 20kV, first grid 80% of target voltage, delayed extraction 125ns. Interpretation of glycan structures corresponding to monoisotopic masses was performed using EXPAZY GlycoMod tool.

Antibody dependent cell cytoxicity Assay (ADCC): Primary B-CLL cells or B-cell lines (Raji) (Target cells) were loaded with 12.5 µM Calcein-AM dye (Sigma, France). 5 000 target cells were the pre-incubated with different concentration of interest MAbs and controls for 20 min at +4°C. Effector cells were then added to the target cells at the ratio E/T equal to 50:1. Specific lysis was calculated using the formula above: (experimental release - (spontaneous release Target + Effector)) / (maximal release - spontaneous release target) * 100, where target and effector cells without antibody represented spontaneous release. Maximal release value was obtained by treating target cells with Triton X-1 00.

Complement dependent cytoxicity Assay (CDC): Target cells (50 000 cells per well) either primary B-CLL cells or B-cell lines (Raji, and Daudi cell lines) were incubated with various MAbs concentration. Then, human normal serum were added the culture and then cells were incubated 4 hours at +37°C under shaking condition. At the end of incubation, lactate deshydrogenase present in supernatant was measured with LDH assay kit (Promega, France). Fluorescence was recorded at the 590 nm excitation wavelength. Specific lysis was calculated using the formula above: (experimental release - target spontaneous release) /(maximal release - target spontaneous release) * 100, where target and effector cells without antibody represented spontaneous release. Maximal release value was obtained by treating target cells with Triton X-1 00.

**RESULTS**

1. Characterization of the glycosylation pattern of the native chR005-1 Fc0 produced by different types of wild type CHO cells.

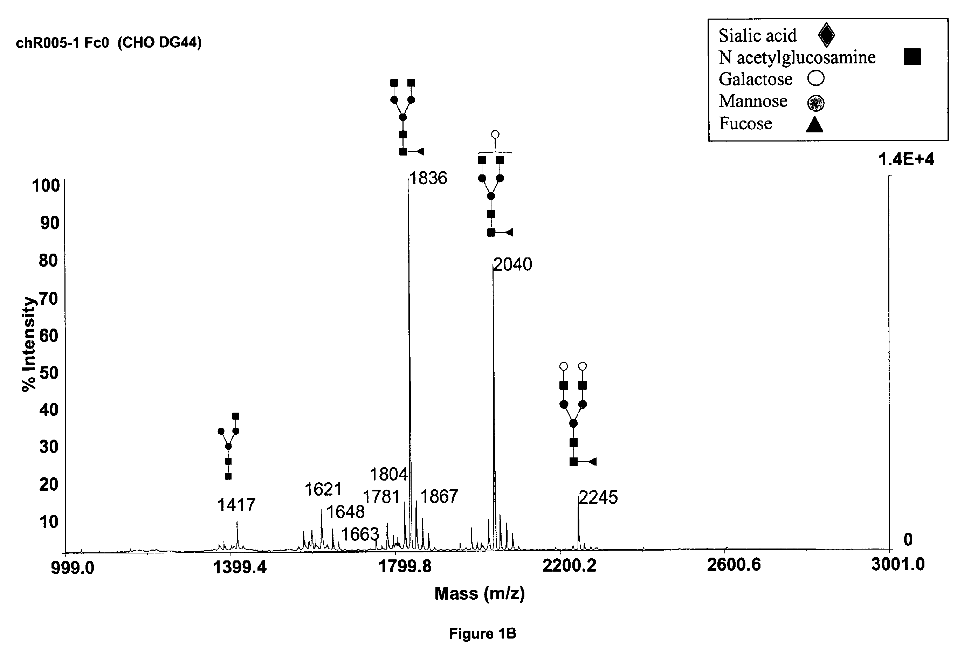

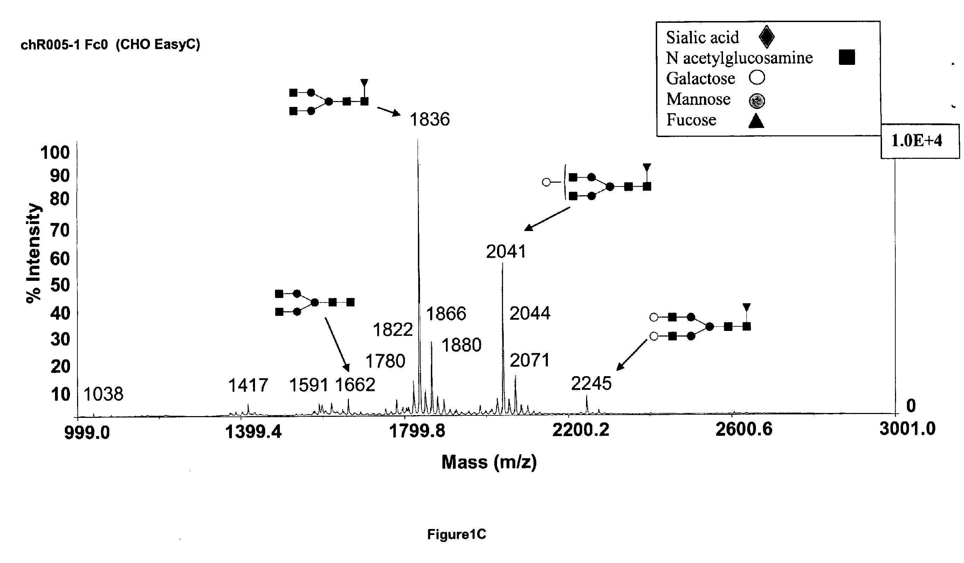

The sugar core found in the Fc region of IgG is a bi-antennary complex [Asn297-GN-GN-M-(M-GN)2] where GN is N-acetylglucosamine, and M is mannose. Oligosaccharides can contain zero (G0), one (G1) or two (G2) galactose (G). Variations of IgG glycosylation patterns can include core fucosylation (F). As shown in Figure 1-3, the three major peaks in the chR005-1 Fc0 sample correspond to masses of fucosylated oligosaccharides with (GlcNAc)2 (Fuc)1 + (Man)3 (GlcNAc)2 (m/z 1836), (Gal)1 (GlcNAc)2 (Fuc)1 + (Man)3(GlcNAc)2 (m/z 2040) and (Gal)2 (GlcNAc)2 (Fuc)1 + (Man)3(GlcNAc)2 (m/z 2245). Other typical structure of N-linked oligosacchararide on IgG was also observed, characterized by a mannosyl-chitobiose core (Man3-GlcNac2-Asn) with or without bisecting GlcNac/L-Fucose (Fuc) and other chain variants including the presence or absence of Galactose (Gal) and sialic acid. In addition, oligosaccharides may contain zero (GO), one (G1) or two (G2) Gal. No significant variation was observed whatever the type of CHO cells (CHO dhfr-/-, CHO DG44, CHO Easy).

2. Cytotoxicity activity characterization of the native chR005-1 Fc0.

In many applications, chimeric antibodies have demonstrated improved effector function in complement-mediated tumor cells lysis and in antibody-dependent cellular cytotoxicity assays as compared to the parental murine monoclonal antibody (Liu *et al.,* 1987; Nishimura *et al.,* 1987; Hamada *et al.,* 1990). The native chimeric MAb chR005-1 Fc0 induced modest ADCC against Burkitt's lymphoma cell line (Figure 4) whatever the CHO cells used for MAb production.

In a standard cytotoxicity assay complement dependent, only rituximab used as positive control killed the Raji cells, whereas the chR005-1 Fc0 failed to trigger cell cytotoxicity whatever the CHO cells used for MAb production (Figure 5).

3. The kifunensine, a potent inhibitor of the glycoprotein processing mannosidase I did not influence the cytotoxic activity of the native chR005-1 Fc0 produced by different types of CHO cells.

When kifunensine was placed in the incubation medium at concentrations of 1 µg/ml, it caused a complete shift in the structure of the N-linked oligosaccharides from complex chains to Man9 (GlcNac)2 (m/z 2396.14) structures in keeping with its inhibition of mannosiadase (Figure 6-8). After kifunensine treatment, the peak corresponding at Man9 (GlcNac)2 (m/z 2396.14) was the major peak compared to untreated chR005-1 Fc0 (71.4% versus 0% respectively). On the other hand, compared to untreated chR005-1 Fc0, the kifunensine had also a strong inhibitory effect on fucosylated oligosaccharides G0F (m/z 1835.93) (36.9% versus 2.8%), G1F (m/z 2040) (35.5% versus 0%) and G2F (m/z 2245) (6.6% versus 0%).

On the other hand, the presence of kifunensine did not impact on the cytotoxicic activity of the native chR005-1 Fc0 such as ADCC (Figure 9) or CDC (Figure 10).

4. Generation of variants for human IgG1 C_{H}2 domain.

As used herein, the term "heavy chain" is used to define the heavy chain of an IgG antibody. In an intact, native IgG, the heavy chain comprises the immunoglobulin domains VH, CH1, Hinge, CH2 and CH3. Throughout the present specification, the numbering of the residues in an IgG heavy chain is that of the EU index as in Kabat *et al,* (1991), expressly incorporated herein by references. The "EU index as in Kabat®" refers to the numbering of the human IgG1 EU antibody.

We constructed several variants including single, double, three, four or five, six or seven substitution variants to enhance ability to mediate effector function, (Figure 11).

5. Comparison of glycosylation profiles among Fc variant antibody panel produced in the wild type CHO Easy cells

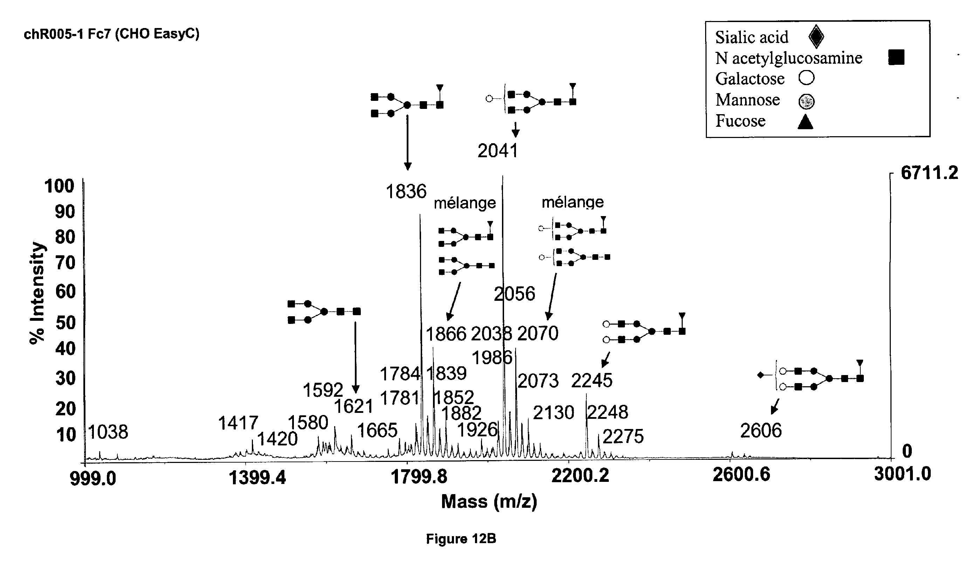

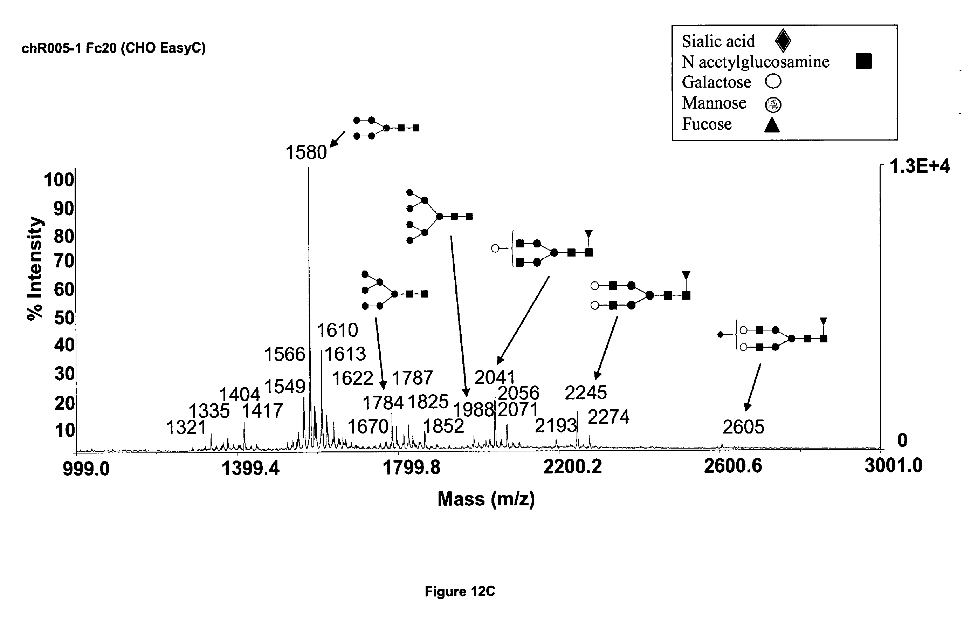

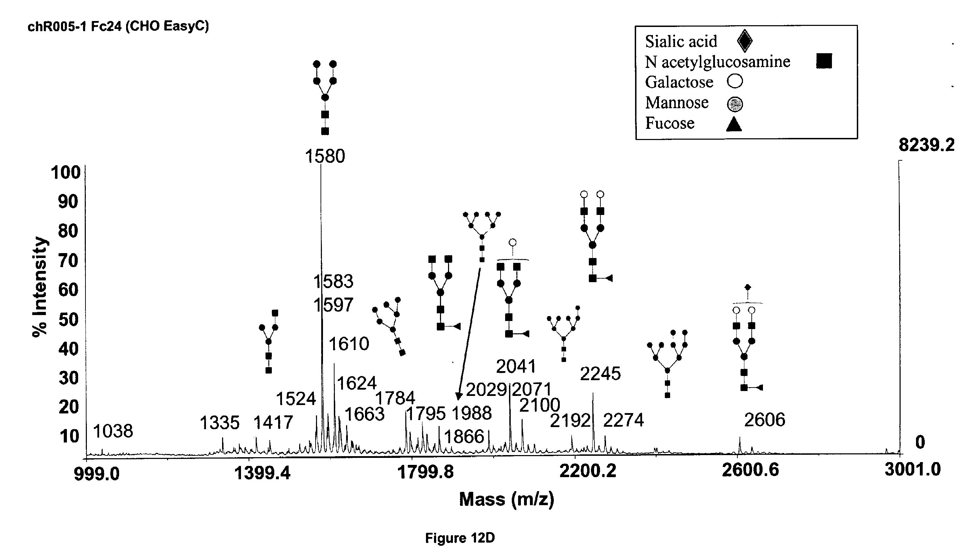

As shown in Figure 12-14, structural differences were observed among the chimeric R005-1 variant antibody panel. Firstly the peak G0F (m/z 1835.93) decreased in the chR005-1 Fc7 (26.2%) and was present at much lower levels in the chR005-1 Fc20 or Fc24 variant antibody (2.1 % and 2.3% respectively) compared to the chimeric R005-1 Fc0 (55.4%).

Whereas the peak G2F (m/z 2040.03) did not decreased in the chR005-1 Fc7 (31.2%), it was present at much lower levels in the chR005-1 Fc20 or Fc24 variant antibody (7.6% and 9% respectively) compared to the chimeric R005-1 Fc0 (34.1%), (Figure 12-14).

Thirdly no or very low impact was observed on the peak G2F (m/z 2244.12), (Figure 12-14.)

Another major difference was the higher level of oligomannoses between (Man)₄(GlcNAc)₂ (m/z 1375.69) to (Man)8(GlcNAc)2 (m/z 2192.08) observed in the chR005-1 Fc20 or Fc24 variant antibody (54.1 % and 50.6% respectively) compared to the wild type chR005-1 Fc0 (0.6%). By contrast this modification was not observed with the chR005-1 Fc7 variant (1.4%), (Figure 12-14).

Another difference was the presence of sialylated glycoforms including (Gal)1 (GlcNAc)₂ (Fuc)₁ (NeuAc)₁ + (Man)₃(GlcNAc)₂- (m/z 2401), (Gal)2 (GlcNAc)₂ (Fuc)₁ (NeuAc)₁ + (Man)₃(GlcNAc)₂- (m/z 2605) and (Gal)3 (GlcNAc)₂ (Fuc)₁ (NeuAc)₁ + (Man)₃(GlcNAc)₂ (m/z 2966) in the chR005-1 Fc7, Fc20 or Fc24 variant antibody (0.9%, 0.6% and 2.4% respectively) compared to the wild type chR005-1 Fc0 (0%), (Figure 12-14).

6. The Fc variants antibody with improved glycosylation profils also influenced the MAb triggered cytotoxicity.

The MAb activity to mediate ADCC from the MAb variant panel was measured using Raji target cells in whole blood based assays (Figure 15). The MAb variant with non fucosylated glycoforms such as the chR005-1 Fc7, Fc20 or Fc24 exhibited a higher level ADCC activity compared to the chR005-1 Fc0 with the highest activity with the chR005-1 Fc24. A strong correlation between ADCC potency and improved glycosylation prolfiles was established. Moreover these data suggest that a strong ADCC could be related to different improved glycosylation antibody profiles.

The MAb activity to mediate CDC from the MAb variant panel was measured using Raji target cells (Figure 16). No complement dependent cytotoxicity was observed with the chR005-1 Fc0 or chR005-1 Fc7 MAb. Although exhibiting a very close glycosylation profile, only the variant chR005-1 Fc24 MAb triggered cytotoxicity complement dependent compared to the chR005-1 Fc20. These data suggest a strong correlation between CDC potency and amino acid sequences.

7. Similar impact of amino acid mutation on Fc was observed other types of wild type CHO cells.

As another example, the chR005-1 Fc20 variant was produced from another wild type CHO cells such as CHO DG44. As shown in Figure 17-19, the peaks G0F (m/z 1835.93) and G1 F (m/z 2040.03) were also present at much lower levels in the chR005-1 Fc20 variant antibody (5.4% and 12.6% respectively) compared to the chimeric R005-1 Fc0 (43.6% and 34.5% respectively). No impact was observed on the peak G2F (m/z 2244.12). Another difference was the higher level of oligomannoses between (Man)₄(GlcNAc)₂ (m/z 1375.69) to (Man)8(GlcNAc)2 (m/z 2192.08) observed in the chR005-1 Fc20 variant antibody (42.3%) compared to the wild type chR005-1 Fc0 (0%).

As another example, the chR005-1 Fc24 variant was produced from another wild such as CHO dhfr-/-. As shown in Figure 20-22, the peaks G0F (m/z 1835.93) and G1 F (m/z 2040.03) were also present at much lower levels in the chR005-1 Fc24 variant antibody (7.2% and 16.8% respectively) compared to the chimeric R005-1 Fc0 (28% and 38.3%). No impact was observed on the peak G2F (m/z 2244.12). Another difference was the higher level of oligomannoses between (Man)₄(GlcNAc)₂ (m/z 1375.69) to (Man)8(GlcNAc)2 (m/z 2192.08) observed in the chR005-1 Fc24 variant antibody (30.5%) compared to the wild type chR005-1 Fc0 (0%).

As another example, the chR005-1 Fc34 variant was produced from another wild such as CHO dhfr-/-. As shown in Figure 23-25, the peaks G0F (m/z 1835.93) and G1 F(m/z 2040.03) were also present at much lower levels in the chR005-1 Fc34 variant antibody (5.2% and 16.1% respectively) compared to the chimeric R005-1 Fc0 (28.0% and 38.3%). No impact was observed on the peak G2F (m/z 2244.12). Another difference was the higher level of oligomannoses between (Man)₄(GlcNAc)₂ (m/z 1375.69) to (Man)8(GlcNAc)2 (m/z 2192.08) observed in the chR005-1 Fc34 variant antibody (29.3%) compared to the wild type chR005-1 Fc0 (0%).

### REFERENCES

Anthony RM, Ravetch JV. A novel role for the IgG Fc glycan: the anti-inflammatory activity of sialylated IgG Fcs. 2010 May. J Clin Immunol. 30 Suppl 1:S9-14.
Bargou R, Leo E, Zugmaier G, et al. Tumor regression in cancer patients by very low doses of a T cell-engaging antibody. 2008. Science. 321:974-7.
Bell AC, Felsenfeld G. Stopped at the border: boundaries and insulators. Curr Opin Genet Dev. 1999 Apr;9(2):191-8.
Boyd PN, Lines AC, Patel AK. The effect of the removal of sialic acid, galactose and total carbohydrate on the functional activity of Campath-1H. 1995 Dec. Mol Immunol. 32(17-18):1311-8.
Bruenke J, Barbin K, Kunert S, Lang P, Pfeiffer M, Stieglmaier K, Niethammer D, Stockmeyer B, Peipp M, Repp R, Valerius T, Fey GH. Effective lysis of lymphoma cells with a stabilised bispecific single-chain Fv antibody against CD19 and Fc gamma RIII (CD16). 2005. Br J Haematol. 130(2):218-28.
Cartron G, Dacheux L, Salles G, Solal-Celigny P, Bardos P, Colombat P, Watier H. Therapeutic activity of humanized anti-CD20 monoclonal antibody and polymorphism in IgG Fc receptor FcgammaRIIIa gene. Blood. 2002 Feb 1 ;99(3):754-8
Ciucanu,I. & Kerek, F. A simple and rapid method for the permethylation of carbohydrates. 1984. Carbohydr. Res. 131, 209-217.
Davies J., L. Jiang, L.Z. Pan, M.J. LaBarre, D. Anderson and M. Reff. Expression of GnTIII in a recombinant anti-CD20 CHO production cell line: expression of antibodies with altered glycoforms leads to an increase in ADCC through higher affinity for FC gamma RIII. 2001. Biotechnol Bioeng. 74, pp. 288-294.
Hallek M, Cheson BD, Catovsky D, Caligaris-Cappio F, Dighiero G, Döhner H, Hillmen P, Keating MJ, Montserrat E, Rai KR, Kipps TJ; International Workshop on Chronic Lymphocytic Leukemia. Guidelines for the diagnosis and treatment of chronic lymphocytic leukemia: a report from the International Workshop on Chronic Lymphocytic Leukaemia updating the National Cancer Institute-Working Group 1996 guidelines. 2008 Dec. Blood. 15;112(13):5259. PMID: 18216293.
Hamada H, Miura K, Ariyoshi K, Heike Y, Sato S, Kameyama K, Kurosawa Y, Tsuruo T. Mouse-human chimeric antibody against the multidrug transporter P-glycoprotein. 1990. Cancer Res. 50(11):3167-71.
Hart CM, Laemmli UK. Facilitation of chromatin dynamics by SARs. Curr Opin Genet Dev. 1998 Oct;8(5):519-25.
Jefferis R, Lund J, Miz+utani H, Nakagawa H, Kawazoe Y, Arata Y, Takahashi N. A comparative study of the N-linked oligosaccharide structures of human IgG subclass proteins. 1990 Jun. Biochem J. 15;268(3):529-37.
Jenkins N, Parekh RB, James DC. Getting the glycosylation right: implications for the biotechnology industry. 1996 Aug. Nat Biotechnol..14(8):975-81.
Kabat EA, Wu TT. Identical V region amino acid sequences and segments of sequences in antibodies of different specificities. Relative contributions of VH and VL genes, minigenes, and complementarity-determining regions to binding of antibody-combining sites. 1991. J Immunol. 147(5):1709-19.
Kabat EA, Wu TT, Reid-Miller M, Perry HM, Gottesman KS, Foeller C. Sequences of proteins of immunological interest. 1991. Maryland: US Department of health and human services, NIH.
Katsutaka Nagai, Senichiro Hakomori and Akira Kobata. 1993. Glycobiology series 2 - Destiny of Sugar Chain in Cell.
Köhler G, Milstein C. Continuous cultures of fused cells secreting antibody of predefined specificity. 1975 Aug. Nature. 7;256(5517):495-7.
Krapp S., Y. Mimura, R. Jefferis, R. Huber and P. Sondermann. Structural analysis of human IgG-Fc glycoforms reveals a correlation between glycosylation and structural integrity. 2003. J Mol Biol 325, pp. 979-989.
Kyte J, Doolittle RF. A simple method for displaying the hydropathic character of a protein. 1982 May. J Mol Biol. 5;157(1):105-32.
Kumpel BM, Rademacher TW, Rook GA, Williams PJ, Wilson IB. Galactosylation of human IgG monoclonal anti-D produced by EBV-transformed B-lymphoblastoid cell lines is dependent on culture method and affects Fc receptor-mediated functional activity. 1994. Hum Antibodies Hybridomas. 5(3-4):143-51.PMID: 7756579.
Kumpel BM, Wang Y, Griffiths HL, Hadley AG, Rook GA. The biological activity of human monoclonal IgG anti-D is reduced by beta-galactosidase treatment. 1995 Hum Antibodies Hybridomas. 6(3):82-8.
Lazar G.A., W. Dang and S. Karki et al.. Engineered antibody Fc variants with enhanced effector function. 2006. Proc Natl Acad Sci USA 103, pp. 4005-4010.
Leatherbarrow RJ, Dwek RA. The effect of aglycosylation on the binding of mouse IgG to staphylococcal protein A. 1990 Nov. Mol Immunol. 27(11):1145-53.
Lifely MR, Hale C, Boyce S, Keen MJ, Phillips J. Glycosylation and biological activity of CAMPATH-1H expressed in different cell lines and grown under different culture conditions. 1995 Dec. Glycobiology. 5(8):813-22.PMID: 8720080.
Liu AY, Robinson RR, Hellström KE, Murray ED Jr, Chang CP, Hellström I. Chimeric mouse-human IgG1 antibody that can mediate lysis of cancer cells. 1987. Proc Natl Acad Sci USA. 84(10):3439-43.
Liu AY, Robinson RR, Murray ED Jr, Ledbetter JA, Hellström I, Hellström KE. Production of a mouse-human chimeric monoclonal antibody to CD20 with potent Fc-dependent biologic activity. 1987. J Immunol. 139(10):3521-6
Lund J, Tanaka T, Takahashi N, Sarmay G, Arata Y, Jefferis R. A protein structural change in aglycosylated IgG3 correlates with loss of huFc gamma R1 and huFc gamma R111 binding and/or activation. 1995 Dec. Mol Immunol. 32(17-18):1311-8.
Mølhøj M, Crommer S, Brischwein K, Rau D, Sriskandarajah M, Hoffmann P, Kufer P, Hofmeister R, Baeuerle PA. CD19-/CD3-bispecific antibody of the BiTE class is far superior to tandem diabody with respect to redirected tumor cell lysis. 2007. Mol Immunol. 44(8):1935-43.
Morelle, W & Michalski J-C. Analysis of protein glycosylation by mass spectrometry. 2007. Nature protocols, vol 2, N °7, 1585-1602.
Mori K., R. Kuni-Kamochi and N. Yamane-Ohnuki et al. Engineering Chinese hamster ovary cells to maximize effector function of produced antibodies using FUT8 siRNA. 2004. Biotechnol Bioeng. 88, pp. 901-908.
Nishimura Y, Yokoyama M, Araki K, Ueda R, Kudo A, Watanabe T. Recombinant human-mouse chimeric monoclonal antibody specific for common acute lymphocytic leukemia antigen. 1987. Cancer Res. 47(4):999-1005.
Niwa R., M. Sakurada and Y. Kobayashi et al. Enhanced natural killer cell binding and activation by low-fucose IgG1 antibody results in potent antibody-dependent cellular cytotoxicity induction at lower antigen density. 2005. Clin Cancer Res 11, pp. 2327-2336.
Radaev S., S. Motyka, W.H. Fridman, C. Sautes-Fridman and P.D. Sun. The structure of a human type III Fcgamma receptor in complex with Fc. 2001. J Biol Chem. 276, pp. 16469-16477.
Radaev S. and P.D. Sun. Recognition of IgG by Fc gamma receptor. The role of Fc glycosylation and the binding of peptide inhibitors. 2001. J Biol Chem. 276, pp. 16478-16483.
Rowland AJ, Pietersz GA, McKenzie IF. Preclinical investigation of the antitumour effects of anti-CD19-idarubicin immunoconjugates. 1993. Cancer Immunol Immunother. 37(3):195-202.
Sapra P, Allen TM. Internalizing antibodies are necessary for improved therapeutic efficacy of antibody- targeted liposomal drugs. 2002. Cancer Res 62: 7190-4.
Sarkar G, Sommer SS. The "megaprimer" method of site-directed mutagenesis. 1990. Biotechniques. 8(4):404-7.
Shields R.L, A.K. Namenuk and K. Hong et al., High resolution mapping of the binding site on human IgG1 for Fc gamma RI, Fc gamma RII, Fc gamma RIII, and FcRn and design of IgG1 variants with improved binding to the Fc gamma R. 2001. J Biol Chem. 276, pp. 6591-6604.
Shields R.L., J. Lai and R. Keck et al. Lack of fucose on human IgG1 N-linked oligosaccharide improves binding to human Fc gamma RIII and antibody-dependent cellular toxicity. 2002. J Biol Chem. 277, pp. 26733-26740.
Shinkawa T., K. Nakamura and N. Yamane et al. The absence of fucose but not the presence of galactose or bisecting N-acetylglucosamine of human IgG1 complex-type oligosaccharides shows the critical role of enhancing antibody-dependent cellular cytotoxicity. 2003. J Biol Chem 278, pp. 3466-3473.
Siberil., C. de Romeuf and N. Bihoreau et al. Selection of a human anti-RhD monoclonal antibody for therapeutic use: impact of IgG glycosylation on activating and inhibitory Fc gamma R functions. 2006. Clin Immuno. 118, pp. 170-179.
Umana P, J. Jean-Mairet, R. Moudry, H. Amstutz and J.E. Bailey. Engineered glycoforms of an antineuroblastoma IgG1 with optimized antibody-dependent cellular cytotoxic activity. 1999. Nat Biotechnol. 17, pp. 176-180.
Yamane-Ohnuki N., S. Kinoshita and M. Inoue-Urakubo et al.. Establishment of FUT8 knockout Chinese hamster ovary cells: an ideal host cell line for producing completely defucosylated antibodies with enhanced antibody-dependent cellular cytotoxicity. 2004. Biotechnol Bioeng. 87, pp. 614-622.

## Claims

1. Use of mutations within the nucleic acid sequence encoding an IgG Fc region to produce an Fc region having a low fucose level and/or a high oligomannose level and/or high level of sialylated glycoforms.

2. The use of claim 1, wherein said nucleic acid is used to produce an antibody containing said Fc.

3. The use of claim 1 or 2, wherein said nucleic acid is used to produce a low fucose level and/or a high oligomannose level and/or high level of sialylated glycoforms Fc in mammal cells, preferably rodent cells, more preferably CHO cells.

4. The use of claims 3, wherein the cells are wild-type cells.

5. The use according to any one of the preceding claims, comprising engineering or using a nucleic acid sequence coding for a variant Fc region wherein this variant region comprises one or several amino acid substitutions at the amino acid positions 243, 292, 300, 305, 326, 333 and 396 of the human IgG Fc region.

6. The use of any one of the preceding claims, wherein the proportion of non-fucosylated Fc or antibodies represent at least 20%, preferably at least 40%, preferably at least 60%" more preferably at least 80% or at a higher level of the Fc or antibodies and/or the proportion Fc or antibodies featured by a higher level of oligomannoses represent at least 20%, preferably at least 30%, more preferably at least 40%, still more preferably at least 50% or at a higher level of the Fc or antibodies and/or the proportion of Fc or antibodies featured by a higher level of sialylated glycoforms represent at least 1.5%, preferably at least 2.5 %, more preferably at least 5% of the Fc or antibodies or at a higher level

7. The use of any one of the preceding claims, wherein the Fc region has a N-linked oligosaccharide structure of a biantennary-type that comprises long chains with terminal GlcNac that are galactosylated.

8. A method to produce an Fc having a low fucose level and/or a high oligomannose level and/or high level of sialylated glycoforms I, comprising engineering one or several variant IgG Fc region(s) comprising one or several mutations, measuring the fucose level present on the Fc region and recovering Fc region(s) having a low fucose level.

9. The method of claim 8, comprising the engineering of a nucleic acid encoding the variant Fc region, the cloning of this nucleic acid in an expression vector, the transfection of mammal cells, preferably rodent cells, more preferably CHO cells, with this expression vector, the recovery of the Fc region having a low fucose level and/or a high oligomannose level and/or high level of sialylated glycoforms.

10. The method of claim 9, wherein the cells are wild-type cells.

11. The method of any one of claims 8-10, wherein upon engineering the mutation in the Fc region, one selects one or several amino acid substitutions at the amino acid positions 243, 292, 300, 305, 326, 333 and 396 of the human IgG Fc region.

12. The method of any one of claims 8-11, wherein the Fc which is produced is part of an antibody containing this Fc, the method comprises recovering the antibody or the antibodies having a low fucose level and/or a high oligomannose level and/or high level of sialylated glycoforms and preferably ADCC and/or CDC function.

13. An antibody obtained by performing the method of any one of claims 8-12.
